# EUROPÄISCHE PATENTANMELDUNG

(11) **EP 4 253 499 A2**
(43) Veröffentlichungstag der Anmeldung: **04.10.2023**
(21) Anmeldenummer: 23181909.5
(22) Anmeldetag: 20.12.2016
(51) Int. Cl.: C09J 175/04

(54) **HYDROXYALDIMIN UND HÄRTBARE POLYURETHANZUSAMMENSETZUNG MIT NIEDRIGEM GEHALT AN MONOMEREN ISOCYANATEN**

(30) Priorität: 21.12.2015 EP 15201650
(62) Teilanmeldung aus: 16820246.3
(71) Anmelder: Sika Technology AG, 6340 Baar (CH)
(72) Erfinder: BURCKHARDT, Urs, 8049 Zürich (CH); KRAMER, Andreas, 8008 Zürich (CH); SCHMIDER, Martin, 21335 Lüneburg (DE)
(74) Vertreter: Sika Patent Attorneys

(57) **Zusammenfassung**

Die vorliegende Erfindung betrifft Hydroxyaldimine der Formel (I), deren Umsetzungsprodukte, insbesondere mit Polyisocyanaten, sowie Isocyanatgruppen aufweisende Zusammensetzungen enthaltend solche Umsetzungsprodukte. Das Hydroxyaldimin ermöglicht Zusammensetzungen mit guter Lagerstabilität, langer Offenzeit, geringem Geruch, schneller, blasenfreier Aushärtung und guten mechanischen Eigenschaften, welche nicht zu Problemen mit Weichmacher-Migration neigen und einen besonders niedrigen Gehalt an monomeren Diisocyanaten aufweisen. Insbesondere ermöglicht es reaktive Heissschmelzklebstoffe mit einer besonders guten Verarbeitbarkeit.

## Beschreibung

### Technisches Gebiet

Die Erfindung betrifft Aldimine und Polyurethane, sowie Klebstoffe, Dichtstoffe und Beschichtungen.

### Stand der Technik

Härtbare Polyurethanzusammensetzungen, welche durch Reaktion von Isocyanatgruppen mit Hydroxylgruppen und/oder Feuchtigkeit bzw. Wasser vernetzen, werden in vielen technischen Anwendungen eingesetzt, beispielsweise als Klebstoffe, Dichtstoffe oder Beschichtungen in der Bau- und Fertigungsindustrie. Die üblichen Produkte weisen einen beträchtlichen Gehalt an monomeren Isocyanaten auf. Dies gilt insbesondere für zweikomponentige Systeme, in welchen die Isocyanat-Komponente zu einem grossen Teil aus monomeren Diisocyanaten besteht. Aber auch einkomponentige Systeme, die auf sogenannten Prepolymeren beruhen, welche Additionsprodukte von monomeren Diisocyanaten mit Polyolen darstellen, enthalten typischerweise einen unerwünscht hohen Gehalt an monomeren Diisocyanaten. Da die Addition relativ unselektiv verläuft und es zu Kettenverlängerung kommt, verbleiben unreagierte monomere Diisocyanate in solchen Produkten. Monomere Diisocyanate sind gesundheitsschädliche Verbindungen, welche unter bestimmten Anwendungsbedingungen aus den Produkten entweichen und für den Verarbeiter aufgrund ihrer Atemwegs-reizenden bzw. -sensibilisierenden Wirkung eine Gefahr darstellen können und deshalb aus Sicht der Arbeitssicherheit zunehmend unerwünscht sind. Dies gilt insbesondere für Spritzapplikationen sowie für heiss zu verarbeitende Zusammensetzungen, wie beispielsweise Heissschmelzklebstoffe.

Es sind verschiedene Wege beschrieben worden, den Anteil an monomeren Diisocyanaten in Polyurethan-Prepolymeren oder Polyurethan-Zusammensetzungen zu senken. So können die monomeren Diisocyanate physikalisch entfernt werden, beispielsweise durch Extraktion oder Destillation, was aber aufwendig und deshalb kostspielig ist. Ein niedriges NCO/OH-Verhältnis bei der Herstellung der Prepolymere führt ebenfalls zu einem niedrigen Gehalt an monomeren Diisocyanaten; so hergestellte Prepolymere weisen aber aufgrund der stärker ausgeprägten Kettenverlängerung eine deutlich erhöhte Viskosität auf und sind damit im Allgemeinen schlechter zu verarbeiten und weniger lagerstabil. Die Verwendung von asymmetrischen Diisocyanaten mit zwei unterschiedlich reaktiven Isocyanatgruppen, wie beispielsweise beschrieben bei WO 03/006521, führt ebenfalls zu einem niedrigen Gehalt an monomeren Diisocyanaten. Solche Prepolymere härten aber langsam aus, da für die Vernetzung nur die weniger reaktive der beiden Isocyanatgruppen zur Verfügung steht.

WO 2007/036575 beschreibt einen Polyurethan-Heissschmelzklebstoff mit niedrigem Isocyanat-Monomergehalt und ein Verfahren zur Verminderung des Gehalts an monomeren Diisocyanaten, indem diese mit speziellen langkettigen Ester-Aldiminen, welche eine Reaktivgruppe mit aktivem Wasserstoff enthalten, umgesetzt werden. Die offenbarten Aldimine und ihre Umsetzungsproduke sind aber temperaturempfindlich, und die damit erhaltenen Heissschmelzklebstoffe weisen eine eingeschränkte Haltbarkeit auf, wenn sie erhitzt werden. Bei der typischen Applikation solcher Heissschmelzklebstoffe über offene Walzen kommt es deshalb bald zu Andickungen und Anhärtungen auf der Walze, die zum Anlagestillstand führen und eine aufwendige Reinigung erfordern können. WO 2008/116927 beschreibt ebenfalls ein Verfahren zur Herstellung einer Polyurethan-Zusammensetzung mit niedrigem Isocyanat-Monomergehalt, in dem die Zusammensetzung mit einem blockierten Amin enthaltend eine Reaktivgruppe mit aktivem Wasserstoff umgesetzt wird. Die ausgeführten Aldimine verursachen aber intensive Geruchsimmissionen durch die freigesetzten leichtflüchtigen Blockierungsmittel und führen teilweise zu geringen Lagerstabilitäten und unerwünscht kurzen Offenzeiten der damit formulierten Zusammensetzungen. Für eine Verwendung als Heissschmelzklebstoffe, welche bei hoher Temperatur typischerweise über offene, der Luft ausgesetzte Walzen verarbeitet werden, sind sie völlig ungeeignet.

### Darstellung der Erfindung

Aufgabe der vorliegenden Erfindung ist es daher, einen Weg zu härtbaren Polyurethanen mit niedrigem Gehalt an monomeren Diisocyanaten bereitzustellen, welcher die Nachteile des Standes der Technik überwindet. Überraschenderweise wurde gefunden, dass ein Hydroxyaldimin der Formel (I) wie in Anspruch 1 beschrieben diese Aufgabe löst. Das Hydroxyaldimin der Formel (I) ist geruchlos, pH-neutral, bei Raumtemperatur flüssig und niedrigviskos und wenig empfindlich gegenüber Wärme und Feuchtigkeit. Es kann somit einfach gelagert, transportiert, dosiert und verarbeitet werden.

Das Hydroxyaldimin der Formel (I) ist geeignet zur Herstellung von Aldiminogruppen aufweisenden Umsetzungsprodukten, insbesondere indem es unter Ausschluss von Feuchtigkeit mit Polyisocyanaten umgesetzt wird. Dabei kann es den Gehalt an monomeren Diisocyanaten von Isocyanatgruppen-haltigen Polyurethanpolymeren sehr effektiv senken, was aus toxikologischen Gründen sehr vorteilhaft ist. Die erhaltenen Umsetzungsprodukte sind für sich allein und in Isocyanatgruppen-haltigen Zusammensetzungen sehr lagerstabil. Unter Feuchtigkeitseinfluss reagieren ihre Aldiminogruppen relativ langsam, aber trotzdem vollständig und störungsfrei, wobei mittels geeigneter Katalysatoren die Reaktion auch schnell eingestellt werden kann. Dadurch wird eine grosse Bandbreite an Offenzeiten ermöglicht. Der bei der Hydrolyse freigesetzte Aldehyd ist unflüchtig, geruchlos und farblos und bewirkt somit weder Emissionen bzw. Geruchsimmissionen noch Verfärbungen. Er ist in Polyurethanen gut verträglich und neigt kaum zu Weichmacher-Migration. Dies ist überraschend. Aldimine, deren Abspalter ein hohes Molekulargewicht aufweisen, sind naturgemäss besonders kritisch in Bezug auf Weichmachermigration nach der Aushärtung, da die eingesetzte Menge aufgrund des hohen Equivalentgewichts entsprechend hoch ist und somit eine hohe Menge an freigesetztem Aldehyd im ausgehärteten Material verbleibt. Zudem liesse der langkettige hydrophobe Alkyl- bzw. Alkoxy-Substituent, insbesondere bei verzweigter Struktur, im hydrophilen, Wasserstoffbrücken aufweisenden Polymergerüst von Polyurethanen eine eher schlechte Verträglichkeit erwarten.

Besonders geeignet ist das Hydroxyaldimin der Formel (I) bzw. seine Umsetzungsprodukte für die Verwendung in reaktiven Polyurethan-Heissschmelzklebstoffen. Es reduziert deren Gehalt an monomeren Diisocyanaten und ermöglicht besonders gute thermische Stabilitäten. Besonders überraschend ist die hervorragende Stabilität von mit Hydroxyaldimin der Formel (I) hergestellten Heisschmelzklebstoffen im heissen geschmolzenen Zustand, wo bei der Verarbeitung solcher Heissschmelzklebstoffe auf offenen rotierenden Walzen über viele Stunden keine nennenswerte Viskositätserhöhung beobachtet wird. Das Hydroxyaldimin der Formel (I) bzw. seine Umsetzungsprodukte ermöglichen Polyurethanzusammensetzungen mit niedrigem Gehalt an monomeren Diisocyanaten, guter Lagerstabilität und gut handhabbaren Verarbeitungszeiten, welche rasch und vollständig und ohne Blasenbildung oder Geruchsimmissionen vernetzen. Dabei entsteht ein blasenfreies Material mit nichtklebriger Oberfläche und guter Festigkeit, Dehnbarkeit und Beständigkeit, welches nicht zu Problemen mit Weichmacher-Migration wie Ausschwitzen (Bleeding), Substratverfärbung oder Spannungsrissbildung im Substrat neigt.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Hydroxyaldimin der Formel (I), 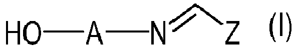 wobei
Z für einen mit einer Alkyl- oder Alkoxy-Gruppe substituierten Aryl-Rest mit insgesamt 12 bis 26 C-Atomen steht, und
A für einen zweiwertigen aliphatischen oder cycloaliphatischen oder arylaliphatischen, gegebenenfalls Ether-Sauerstoff aufweisenden Kohlenwasserstoff-Rest mit einem Molekulargewicht im Bereich von 28 bis 500 g/mol steht.

Eine gestrichelte Linie in den Formeln stellt jeweils die Bindung zwischen einem Substituenten und dem zugehörigen Molekülrest dar.

Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.

Mit "Poly" beginnende Substanznamen wie Polyamin, Polyol oder Polyisocyanat bezeichnen Substanzen, die formal zwei oder mehr der in ihrem Namen vorkommenden funktionellen Gruppen pro Molekül enthalten.

Als "primäres Polyamin" wird eine Verbindung mit mindestens zwei primären Aminogruppen bezeichnet.

Als "aromatisches Isocyanat" wird ein Isocyanat bezeichnet, dessen Isocyanatgruppen direkt an ein aromatisches C-Atom gebunden sind. Dementsprechend werden solche Isocyanatgruppen als "aromatische Isocyanatgruppen" bezeichnet.

Als "Molekulargewicht" wird die molare Masse (in Gramm pro Mol) eines Moleküls oder eines Molekül-Rests bezeichnet. Als "mittleres Molekulargewicht" wird das Zahlenmittel des Molekulargewichts (Mₙ) einer polydispersen Mischung von oligomeren oder polymeren Molekülen oder Molekül-Resten bezeichnet. Es wird üblicherweise mittels Gelpermeationschromatographie (GPC) gegen Polystyrol als Standard bestimmt.

Mit dem Begriff "Viskosität" wird die dynamische Viskosität oder Scherviskosität bezeichnet, welche durch das Verhältnis zwischen der Schubspannung und der Scherrate (Geschwindigkeitsgefälle) definiert ist und wie in DIN EN ISO 3219 beschrieben bestimmt wird.

Als "lagerstabil" oder "lagerfähig" wird eine Substanz oder eine Zusammensetzung bezeichnet, wenn sie bei Raumtemperatur in einem geeigneten Gebinde während längerer Zeit, typischerweise während mindestens 3 Monaten bis zu 6 Monaten und mehr, aufbewahrt werden kann, ohne dass sie sich in ihren Anwendungs- oder Gebrauchseigenschaften durch die Lagerung in einem für ihren Gebrauch relevanten Ausmass verändert.

Als "einkomponentig" wird eine Zusammensetzung bezeichnet, bei welcher alle Bestandteile der Zusammensetzung im gleichen Gebinde vorliegen und welche als solche lagerstabil ist.

Als "zweikomponentig" wird eine Zusammensetzung bezeichnet, bei welcher die Bestandteile der Zusammensetzung in zwei verschiedenen Komponenten vorliegen, welche in voneinander getrennten Gebinden gelagert und erst kurz vor oder während der Applikation der Zusammensetzung miteinander vermischt werden.

Als "Aushärtung" oder "Vernetzung" wird das chemische Abbinden einer härtbaren Zusammensetzung bezeichnet.

Als "Raumtemperatur" wird eine Temperatur von 23°C bezeichnet.

Z steht bevorzugt für einen Rest der Formel (II), 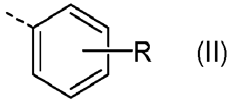
wobei R für einen linearen oder verzweigten Alkyl- oder Alkoxy-Rest mit 6 bis 20, bevorzugt 8 bis 16, C-Atomen steht.
R steht bevorzugt für einen linearen oder verzweigten Alkyl-Rest mit 10 bis 14 C-Atomen oder für einen linearen oder verzweigten Alkoxy-Rest mit 8 bis 12 C-Atomen.
R steht insbesondere für einen linearen oder verzweigten Alkyl-Rest mit 10 bis 14 C-Atomen. Ein solches Hydroxyaldimin ist besonders reaktiv.

R steht besonders bevorzugt für einen verzweigten Alkyl-Rest. Ein solches Hydroxyaldimin ist bei Raumtemperatur typischerweise flüssig und vergleichsweise niedrigviskos, was für seine Handhabung vorteilhaft ist.

Insbesondere steht R für einen verzweigten Alkyl-Rest mit 10 bis 14 C-Atomen.

R steht ganz besonders bevorzugt für einen verzweigten Alkyl-Rest mit 10 bis 14 C-Atomen. Ein solches Hydroxyaldimin ist besonders reaktiv, bei Raumtemperatur meist flüssig und vergleichsweise niedrigviskos.

Bevorzugt steht R in meta- oder para-Stellung, insbesondere in para-Stellung. Ein solches Hydroxyaldimin ist besonders gut zugänglich.

Ganz besonders bevorzugt steht R für einen Rest der Formel 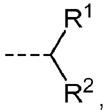 wobei R¹ und R² jeweils für einen Alkyl-Rest stehen und zusammen 9 bis 13 C-Atome aufweisen. Bevorzugt sind die Reste R¹ und R² jeweils linear.

Am meisten bevorzugt steht Z somit für einen Rest der Formel (IIa), 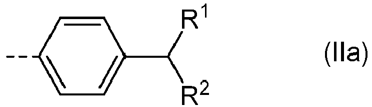 wobei R¹ und R² die genannten Bedeutungen aufweisen.

Die bevorzugten Reste Z sind besonders gut zugänglich und ermöglichen besonders geruchsarme Hydroxyaldimine, welche bei Raumtemperatur insbesondere flüssig und besonders niedrigviskos sind.

A weist bevorzugt ein Molekulargewicht im Bereich von 28 bis 250 g/mol auf.

Besonders bevorzugt steht A für einen Rest ausgewählt aus der Gruppe bestehend aus 1,2-Ethylen, 1,2-Propylen, 1,3-Propylen, 1,5-Pentylen, 1,6-Hexylen, (1,5,5-Trimethylcyclohexan-1-yl)methan-1,3 und 3-Oxa-1,5-pentylen. Davon bevorzugt ist 1,2-Ethylen, 1,5-Pentylen, 1,6-Hexylen, (1,5,5-Trimethylcyclohexan-1-yl)methan-1,3 oder 3-Oxa-1,5-pentylen.

Diese Reste A sind besonders gut zugänglich.

Besonders bevorzugt ist 3-Oxa-1,5-pentylen. Diese Hydroxyaldimine neigen nicht zum Cyclisieren und ermöglichen Isocyanatgruppen aufweisende Zusammensetzungen mit einem besonders niedrigen Gehalt an monomeren Diisocyanaten und Heissschmelzklebstoffe mit besonders guter Stabilität während der Verarbeitung im geschmolzenen Zustand.

Besonders bevorzugt ist weiterhin (1,5,5-Trimethylcyclohexan-1-yl)methan-1,3. Diese Hydroxyaldimine neigen nicht zum Cyclisieren und ermöglichen Isocyanatgruppen aufweisende Zusammensetzungen mit einem besonders niedrigen Gehalt an monomeren Diisocyanaten und mit besonders guter Lagerstabilität.

Die bevorzugten Hydroxyaldimine der Formel (I) sind besonders gut zugänglich und ermöglichen besonders gute Eigenschaften bei der erfindungsgemässen Verwendung.

Das Hydroxyaldimin der Formel (I) steht gegebenenfalls im Gleichgewicht mit einer cyclischen Verbindung der Formel (I'), 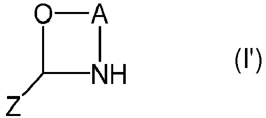 wobei A und Z die bereits genannten Bedeutungen aufweisen. Verbindungen der Formel (I') werden insbesondere für den Fall beobachtet, dass die Aldiminogruppe und die Hydroxylgruppe im Hydroxyaldimin der Formel (I) durch zwei oder drei Kohlenstoffatome getrennt sind; sie stellen dabei Verbindung der Formel (I') 2-substituierte 1,3-Oxazolidine (5-Ring) oder Tetrahydro-1,3-oxazine (6-Ring) dar.

Das Hydroxyaldimin der Formel (I) wird bevorzugt erhalten aus der Umsetzung von mindestens einem Amin der Formel (III) mit mindestens einem Aldehyd der Formel (IV) in einer Kondensationsreaktion unter Freisetzung von Wasser.

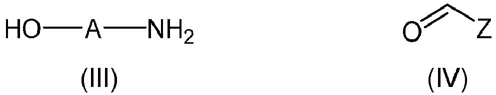

In den Formeln (III) und (IV) weisen A und Z die bereits genannten Bedeutungen auf.

Der Aldehyd der Formel (IV) wird dabei bevorzugt stöchiometrisch oder im stöchiometrischen Überschuss in Bezug auf die primären Aminogruppen eingesetzt. Auf diese Weise ist das Reaktionsprodukt weitgehend oder ganz frei von primären Aminogruppen.

Ein weiterer Gegenstand der Erfindung ist somit ein Reaktionsprodukt enthaltend mindestens ein Hydroxyaldimin der Formel (I), erhalten aus der Umsetzung von mindestens einem Amin der Formel (III) mit mindestens einem Aldehyd der Formel (IV) in einer Kondensationsreaktion unter Freisetzung von Wasser, wobei der Aldehyd stöchiometrisch oder im stöchiometrischen Überschuss in Bezug auf die primären Aminogruppen vorhanden war.

Die Umsetzung wird vorteilhaft bei einer Temperatur im Bereich von 15 bis 120°C, bevorzugt bei 20 bis 100°C, durchgeführt, gegebenenfalls in Anwesenheit eines Lösemittels. Das Kondensationswasser wird bevorzugt aus der Reaktionsmischung entfernt, entweder als Azeotrop mit einem geeigneten Lösemittel oder bevorzugt direkt durch Destillation, gegebenenfalls unter Vakuum.

Gegebenenfalls wird bei der Umsetzung ein Katalysator eingesetzt, insbesondere ein Säure-Katalysator.

Besonders bevorzugt wird ohne Lösemittel gearbeitet und das Kondensationswasser mittels Anlegen von Vakuum aus der erwärmten Reaktionsmischung entfernt.

Ein solches Reaktionsprodukt kann ohne weitere Aufarbeitung als Hydroxyaldimin der Formel (I) verwendet werden.

Bevorzugt wird das Amin der Formel (III) mit dem Aldehyd der Formel (IV) zu einer Reaktionsmischung vereint, wobei der Aldehyd in Bezug auf die primären Aminogruppen stöchiometrisch oder im stöchiometrischen Überschuss vorhanden ist, und das Kondensationswasser mit einer geeigneten Methode aus der Reaktionsmischung entfernt wird, gegebenenfalls unter Erwärmen der Reaktionsmischung.

Als Amin der Formel (III) sind primäre Hydroxyamine geeignet, insbesondere 2-Aminoethanol, 2-Methylaminoethanol (2-Amino-1-propanol), 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethylcyclohexanol, eine primäre Aminogruppe tragende Derivate von Glykolen wie Diethylenglykol, Dipropylenglykol, Dibutylenglykol oder höheren Oligomeren oder Polymeren dieser Glykole, insbesondere 2-(2-Aminoethoxy)ethanol, 2-(2-(2-Aminoethoxy)-ethoxy)ethanol, α-(2-Hydroxymethylethyl)-ω-(2-aminomethylethoxy)poly(oxy-(methyl-1,2-ethandiyl)), eine Hydroxylgruppe und eine primäre Aminogruppe tragende Derivate von polyalkoxylierten drei- oder höherwertigen Alkoholen, Produkte aus der einfachen Cyanoethylierung und anschliessenden Hydrierung von Glykolen, insbesondere 3-(2-Hydroxyethoxy)propylamin, 3-(2-(2-Hydroxyethoxy)ethoxy)propylamin oder 3-(6-Hydroxyhexyloxy)propylamin. Bevorzugt ist 2-Aminoethanol, 2-Methylaminoethanol (2-Amino-1-propanol), 1-Amino-2-propanol, 3-Amino-1-propanol, 5-Amino-1-pentanol, 6-Amino-1-hexanol, 3-Aminomethyl-3,5,5-trimethylcyclohexanol oder 2-(2-Aminoethoxy)ethanol.

Besonders bevorzugt ist 3-Aminomethyl-3,5,5-trimethylcyclohexanol oder 2-(2-Aminoethoxy)ethanol.

Ein bevorzugter Aldehyd der Formel (IV) ist ein Aldehyd der Formel (IVa), wobei R die bereits beschriebenen Bedeutungen aufweist.

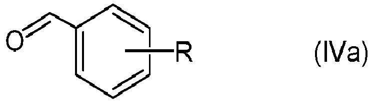

Ein besonders bevorzugter Aldehyd der Formel (IV) ist ein Aldehyd der Formel (IVb), wobei R¹ und R² die bereits beschriebenen Bedeutungen aufweisen.

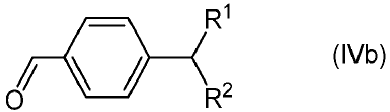

Als Aldehyd der Formel (IV) insbesondere bevorzugt sind 4-Decylbenzaldehyde, 4-Undecylbenzaldehyde, 4-Dodecylbenzaldehyde, 4-Tridecylbenzaldehyde oder 4-Tetradecylbenzaldehyde, bei welchen die Alkyl-Reste linear oder verzweigt, insbesondere verzweigt, sind.

Als Aldehyd der Formel (IV) am meisten bevorzugt ist ein Gemisch enthaltend 4-Decylbenzaldehyde, 4-Undecylbenzaldehyde, 4-Dodecylbenzaldehyde, 4-Tridecylbenzaldehyde und 4-Tetradecylbenzaldehyde, deren Alkyl-Reste mehrheitlich verzeigt sind.

Der Aldehyd der Formel (IV) ist insbesondere erhältlich aus der Formylierung von mindestens einem Alkyl- und/oder Alkoxy-substituierten aromatischen Kohlenwasserstoff mit Kohlenmonoxid unter Einwirkung eines Säurekatalysators. Als Säurekatalysator eignet sich beispielsweise das System HCl-AlCl3 (Gattermann-Koch-Reaktion).

In einem bevorzugten Herstellprozess wird die Formylierung mit HF-BF₃ als Säurekatalysator durchgeführt. Dies ist vorteilhaft, da dieses Verfahren besonders selektiv verläuft und der Aldehyd der Formel (IV) aus dem Reaktionsgemisch ohne Hydrolyseschritt abgetrennt und der Katalysator wiederverwendet werden kann, womit eine aufwendige Produktaufarbeitung und Abfallentsorgung entfällt.

Bevorzugt ist das Hydroxyaldimin der Formel (I) ausgewählt aus der Gruppe bestehend aus N-(4-Alkylbenzyliden)-2-aminoethanol, N-(4-Alkylbenzyliden)-2-methylaminoethanol, N-(4-Alkylbenzyliden)-1-amino-2-propanol, N-(4-Alkylbenzyliden)-3-amino-1-propanol, N-(4-Alkylbenzyliden)-5-amino-1-pentanol, N-(4-Alkylbenzyliden)-6-amino-1-hexanol, N-(4-Alkylbenzyliden)-3-aminomethyl-3,5,5-trimethylcyclohexanol und N-(4-Alkylbenzyliden)-2-(2-aminoethoxy)ethanol, wobei Alkyl jeweils für einen linearen oder insbesondere verzweigten Decyl-, Undecyl-, Dodecyl-, Tridecyl- oder Tetradecyl-Rest steht.

Davon bevorzugt ist N-(4-Alkylbenzyliden)-3-aminomethyl-3,5,5-trimethylcyclo-hexanol oder N-(4-Alkylbenzyliden)-2-(2-aminoethoxy)ethanol.

Bevorzugt stellt das Hydroxyaldimin der Formel (I) ein Gemisch dar aus Hydroxyaldiminen der Formel (I), bei welchen Z jeweils für einen Rest der Formel (II) steht und R dabei ausgewählt ist aus Alkyl-Resten mit 6 bis 20 C-Atomen. Besonders bevorzugt ist R dabei ausgewählt aus linearen oder insbesondere verzweigten Decyl-, Undecyl-, Dodecyl-, Tridecyl- und Tetradecyl-Resten.

Ein weiterer Gegenstand der Erfindung ist somit ein Gemisch aus Hydroxyaldiminen der Formel (I), bei welchen Z jeweils für einen Rest der Formel (II) steht und R dabei ausgewählt ist aus linearen oder insbesondere verzweigten Decyl-, Undecyl-, Dodecyl-, Tridecyl- und Tetradecyl-Resten.

Ein solches Gemisch ist technisch besonders einfach zugänglich.

Das Hydroxyaldimin der Formel (I) kann verwendet werden als Härter für Isocyanatgruppen aufweisende Zusammensetzungen.

Besonders vorteilhaft wird das Hydroxyaldimin der Formel (I) zur Herstellung von Aldiminogruppen aufweisenden Umsetzungsprodukten verwendet, wobei mindestens ein Hydroxyaldimin der Formel (I) mit mindestens einer Verbindung, welche gegenüber Hydroxylgruppen reaktive Gruppen aufweist, umgesetzt wird. Bevorzugt ist die Verbindung in Bezug auf diese Reaktivgruppen mehrfunktionell. Besonders bevorzugt handelt es sich dabei um mindestens ein Polyisocyanat oder um mindestens einen mehrfunktionellen Ester, insbesondere um mindestens ein Polyisocyanat. Solche Aldiminogruppen aufweisende Umsetzungsprodukte können vorteilhaft verwendet werden als latente Härter für Isocyanatgruppen aufweisende Zusammensetzungen.

Die erfindungsgemässe Verwendung zeichnet sich dadurch aus, dass das Hydroxyaldimin der Formel (I) bzw. seine Umsetzungsprodukte ausgezeichnet verträglich sind mit Isocyanatgruppen-haltigen Zusammensetzungen. Insbesondere ermöglichen sie unter Ausschluss von Feuchtigkeit eine gute Lagerstabilität zusammen mit Isocyanatgruppen und bei Kontakt mit Feuchtigkeit eine lange Offenzeit mit einer raschen und vollständigen Aushärtung, ohne dabei Geruchsimmissionen zu verursachen, wobei mechanisch hochwertige und beständige Polymere, die nicht zu Problemen mit Weichmacher-Migration neigen, erhalten werden.

Ein weiterer Gegenstand der Erfindung ist ein Umsetzungsprodukt mit Aldiminogruppen der Formel (V), 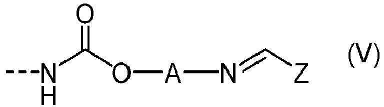 erhalten aus der Umsetzung von mindestens einem Hydroxyaldimin der Formel (I) mit mindestens einem Polyisocyanat.

In der Formel (V) weisen A und Z die bereits genannten Bedeutungen auf.

Für die Umsetzung wird mindestens ein Hydroxyaldimin der Formel (I) unter Ausschluss von Feuchtigkeit mit mindestens einem Polyisocyanat vermischt, worauf die Hydroxylgruppen mit vorhandenen Isocyanatgruppen reagieren. Die Umsetzung kann bei Umgebungstemperatur oder bei einer erhöhten Temperatur erfolgen. Bevorzugt ist eine Temperatur im Bereich von 0 bis 180°C, bevorzugt 10 bis 180°C, insbesondere 20 bis 180°C. Dabei kann ein Katalysator vorhanden sein, insbesondere eine Bismut(III)-, Zink(II)-, Zirkonium(IV)- oder Zinn(II)-Verbindung oder eine Organozinn(IV)-Verbindung. Bei der Umsetzung können weitere Bestandteile von üblicherweise in Polyurethanzusammensetzungen verwendeten Substanzen vorhanden sein, zum Beispiel Füllstoffe, Weichmacher oder Lösemittel.

Die Umsetzung kann unterstöchiometrisch, d.h. bei einem OH/NCO-Verhältnis von unter 1, durchgeführt werden. Dabei wird ein Umsetzungsprodukt erhalten, welches neben Aldiminogruppen zusätzlich auch Isocyanatgruppen aufweist. Ein solches Umsetzungsprodukt ist unter Ausschluss von Feuchtigkeit lagerstabil. Es ist insbesondere geeignet als latenter Härter für Isocyanatgruppen aufweisende Zusammensetzungen oder als Bindemittel in feuchtigkeitshärtenden Polyurethanzusammensetzungen.

In einer besonders bevorzugten Ausführungsform liegt das OH/NCO-Verhältnis bei der Umsetzung im Bereich von 0.05/1 bis 0.5/1, insbesondere 0.1/ bis 0.5/1, bevorzugt 0.2/1 bis 0.5/1. Ein solches Umsetzungsprodukt vernetzt bei Kontakt mit Feuchtigkeit zu einem festen Material. Dabei reagieren die hydrolysierenden Aldiminogruppen mit vorhandenen Isocyanatgruppen und überschüssige Isocyanatgruppen reagieren direkt mit Feuchtigkeit. Für den Fall, dass es sich beim Polyisocyanat um ein Isocyanatgruppen-haltiges Polyurethanpolymer handelt, liegt ein Vorteil des Umsetzungsproduktes darin, dass sein Gehalt an monomeren Diisocyanaten gegenüber dem eingesetzten Polyurethanpolymer stark reduziert ist. Dabei bleibt das Umsetzungsprodukt auf die gleiche Weise verwendbar wie das für die Umsetzung verwendete Polyurethanpolymer, insbesondere als Bindemittel in einkomponentigen feuchtigkeitshärtenden Polyurethanen, ohne dass dabei nennenswerte Nachteile bei Lagerstabilität, Reaktivität, Geruch oder den mechanischen Eigenschaften bestehen.

Die Umsetzung kann weiterhin stöchiometrisch oder überstöchiometisch, d.h. bei einem OH/NCO-Verhältnis von 1 oder darüber, durchgeführt werden. Dabei wird ein Umsetzungsprodukt erhalten, welches frei ist von Isocyanatgruppen. Ein solches Umsetzungsprodukt ist auch in Gegenwart von Feuchtigkeit lagerstabil. Es ist insbesondere geeignet als latenter Härter in ein- oder zweikomponentigen Isocyanatgruppen aufweisenden Zusammensetzungen.

In einer bevorzugten Ausführungsform liegt das OH/NCO-Verhältnis bei der Umsetzung im Bereich von 1/1 bis 1.5/1, insbesondere 1/1 bis 1.2/1, bevorzugt 1/1 bis 1.1/1.

Für die Herstellung von Umsetzungsprodukten mit Aldiminogruppen der Formel (V) geeignete Hydroxyaldimine der Formel (I) sind die vorgängig genannten, insbesondere die bevorzugten Ausführungsformen.

Für die Herstellung von Umsetzungsprodukten mit Aldiminogruppen der Formel (V) geeignete Polyisocyanate sind insbesondere
- aliphatische, cycloaliphatische oder arylaliphatische Di- oder Triisocyanate, bevorzugt 1,4-Tetramethylendiisocyanat, 2-Methylpentamethylen-1,5-diisocyanat, 1,6-Hexamethylendiisocyanat (HDI), 2,2,4- und/oder 2,4,4-Trimethyl-1,6-hexamethylendiisocyanat (TMDI), 1,10-Decamethylendiisocyanat, 1,12-Dodecamethylendiisocyanat, Lysin- oder Lysinesterdiisocyanat, Cyclohexan-1,3- oder -1,4-diisocyanat, 1-Methyl-2,4- und/oder -2,6-diisocyanatocyclohexan (H₆TDI), 1-Isocyanato-3,3,5-trimethyl-5-isocyanatomethylcyclohexan (IPDI), Perhydro-2,4'- und/oder -4,4'-diphenylmethandiisocyanat (H₁₂MDI), 1,3- oder 1,4-Bis-(isocyanatomethyl)cyclohexan, m- oder p-Xylylendiisocyanat, Tetramethyl-1,3- oder -1,4-xylylendiisocyanat, 1,3,5-Tris-(isocyanatomethyl)benzol, Bis-(1-Isocyanato-1-methylethyl)naphthalin, Dimer- oder Trimerfettsäureisocyanate wie insbesondere 3,6-Bis-(9-isocyanatononyl)-4,5-di(1-heptenyl)cyclohexen (Dimeryldiisocyanat); insbesondere H₁₂MDI oder HDI oder IPDI;
- aromatische Di- oder Triisocyanate, bevorzugt 4,4'- oder 2,4'- oder 2,2'-Diphenylmethandiisocyanat oder beliebigen Gemischen dieser Isomeren (MDI), 2,4- oder 2,6-Toluylendiisocyanat oder beliebige Gemische dieser Isomeren (TDI), Gemische aus MDI und MDI-Homologen (polymeres MDI oder PMDI), 1,3- oder 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), Tris-(4-isocyanatophenyl)methan oder Tris-(4-isocyanatophenyl)thiophosphat; insbesondere MDI oder TDI;
- Oligomere oder Derivate der genannten Di- oder Triisocyanate, insbesondere abgeleitet von HDI, IPDI, MDI oder TDI, insbesondere Oligomere enthaltend Uretdion- oder Isocyanurat- oder Iminooxadiazindion-Gruppen oder verschiedene dieser Gruppen; oder zwei- oder mehrwertige Derivate enthaltend Ester- oder Harnstoff- oder Urethan- oder Biuret- oder Allophanat- oder Carbodiimid- oder Uretonimin- oder Oxadiazintrion-Gruppen oder verschiedene dieser Gruppen. Solche Polyisocyanate stellen in der Praxis üblicherweise Gemische von Substanzen mit unterschiedlichen Oligomerisierungsgraden und/oder chemischen Strukturen dar. Insbesondere weisen sie eine mittlere NCO-Funktionalität von 2.1 bis 4.0 auf.
- Isocyanatgruppen-haltige Polyurethanpolymere aus der Umsetzung von Polyolen mit den vorgenannten Polyisocyanaten, wie nachfolgend beschrieben.

Ein geeignetes Isocyanatgruppen-haltiges Polyurethanpolymer wird insbesondere erhalten aus der Umsetzung von mindestens einem Polyol mit einer überstöchiometrischen Menge von mindestens einem Diisocyanat. Die Umsetzung wird bevorzugt unter Feuchtigkeitsausschluss bei einer Temperatur im Bereich von 50 bis 160°C durchgeführt, gegebenenfalls in Anwesenheit geeigneter Katalysatoren. Das NCO/OH-Verhältnis liegt bevorzugt im Bereich von 1.3/1 bis 2.5/1. Das nach der Umsetzung der OH-Gruppen im Reaktionsgemisch verbleibende monomere Diisocyanat kann grösstenteils entfernt werden, insbesondere mittels Destillation, was im Fall eines hohen NCO/OH-Verhältnisses bevorzugt ist. Das erhaltene Polyurethanpolymer weist bevorzugt einen Gehalt an freien Isocyanatgruppen im Bereich von 0.5 bis 10 Gewichts-%, insbesondere 1 bis 5 Gewichts-%, besonders bevorzugt 1 bis 3 Gewichts-%, auf. Bevorzugt weist es einen Restgehalt an monomeren Diisocyanaten im Bereich von 0.5 bis 5 Gewichts-%, insbesondere 1 bis 3 Gewichts-%, auf. Gegebenenfalls kann das Polyurethanpolymer unter Mitverwendung von Weichmachern oder Lösemitteln hergestellt werden, wobei die verwendeten Weichmacher oder Lösemittel keine gegenüber Isocyanaten reaktive Gruppen enthalten.

Das Isocyanatgruppen-haltige Polyurethanpolymer weist bevorzugt ein mittleres Molekulargewicht im Bereich von 500 bis 20'000 g/mol, insbesondere 1'000 bis 15'000 g/mol, bevorzugt 1'500 bis 10'000 g/mol, auf.

Als Polyisocyanat zur Herstellung eines Isocyanatgruppen-haltigen Polyurethanpolymers bevorzugt sind Diisocyanate, insbesondere MDI, TDI, IPDI, HDI oder H₁₂MDI.

Als Polyol zur Herstellung eines Isocyanatgruppen-haltigen Polyurethanpolymers geeignet sind
- Polyetherpolyole, insbesondere Polyoxyalkylendiole und/oder Polyoxyalkylentriole, insbesondere Polymerisationsprodukte von Ethylenoxid oder 1,2-Propylenoxid oder 1,2- oder 2,3-Butylenoxid oder Oxetan oder Tetrahydrofuran oder Mischungen davon, wobei diese mit Hilfe eines Startermoleküls mit zwei oder mehreren aktiven Wasserstoffatomen polymerisiert sein können, insbesondere einem Startermolekül wie Wasser, Ammoniak oder einer Verbindung mit mehreren OH- oder NH-Gruppen wie beispielsweise 1,2-Ethandiol, 1,2- oder 1,3-Propandiol, Neopentylglykol, Diethylenglykol, Triethylenglykol, die isomeren Dipropylenglykole oder Tripropylenglykole, die isomeren Butandiole, Pentandiole, Hexandiole, Heptandiole, Octandiole, Nonandiole, Decandiole, Undecandiole, 1,3- oder 1,4-Cyclohexandimethanol, Bisphenol A, hydriertes Bisphenol A, 1,1,1-Trimethylolethan, 1,1,1-Trimethylolpropan, Glycerin oder Anilin, oder Mischungen der vorgenannten Verbindungen. Ebenfalls geeignet sind Polyetherpolyole mit darin dispergierten Polymerpartikeln, insbesondere solche mit Styrol-Acrylnitril-Partikeln (SAN) oder Polyharnstoff- bzw. Polyhydrazodicarbonamid-Partikeln (PHD). Bevorzugte Polyetherpolyole sind Polyoxypropylendiole oder Polyoxypropylentriole, oder sogenannte Ethylenoxid-terminierte (EO-endcapped) Polyoxypropylendiole oder -triole. Letztere sind Polyoxyethylen-polyoxypropylen-Mischpolyole, die insbesondere dadurch erhalten werden, dass Polyoxypropylendiole oder -triole nach Abschluss der Polypropoxylierungsreaktion mit Ethylenoxid weiter alkoxyliert werden und dadurch schliesslich primäre Hydroxylgruppen aufweisen. Bevorzugte Polyetherpolyole weisen einen Ungesättigtheitsgrad von weniger als 0.02 mEq/g, insbesondere weniger als 0.01 mEq/g auf.
- Polyesterpolyole, auch Oligoesterole genannt, hergestellt nach bekannten Verfahren, insbesondere der Polykondensation von Hydroxycarbonsäuren bzw. Lactonen oder der Polykondensation von aliphatischen und/oder aromatischen Polycarbonsäuren mit zwei- oder mehrwertigen Alkoholen. Bevorzugt sind Polyesterdiole aus der Umsetzung von zweiwertigen Alkoholen wie insbesondere 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole, mit organischen Dicarbonsäuren oder deren Anhydride oder Ester, wie insbesondere Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure oder Hexahydrophthalsäure oder Mischungen der vorgenannten Säuren, oder Polyesterpolyole aus Lactonen wie insbesondere ε-Caprolacton. Besonders bevorzugt sind Polyesterpolyole aus Adipinsäure oder Sebacinsäure oder Dodecandicarbonsäure und Hexandiol oder Neopentylglykol.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.
- Polyacrylat- und Polymethacrylatpolyole.
- Polyhydroxyfunktionelle Fette und Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen und Isopren, beispielsweise polyhydroxyfunktionelle Acrylonitril/ Butadien-Copolymere, wie sie beispielsweise aus Epoxiden oder Aminoalkoholen und carboxylterminierten Acrylonitril/Butadien-Copolymeren (beispielsweise kommerziell erhältlich unter dem Namen Hypro^{®} CTBN oder CTBNX oder ETBN von Emerald Performance Materials) hergestellt werden können; sowie hydrierte polyhydroxyfunktionelle Polymere oder Copolymere von Dienen.

Geeignet sind insbesondere auch Mischungen von Polyolen.

Bevorzugt sind Polyetherpolyole, Polyesterpolyole, Polycarbonatpolyole, Poly-(meth)acrylatpolyole oder Polybutadienpolyole.

Besonders bevorzugt sind Polyetherpolyole, Polyesterpolyole, insbesondere aliphatische Polyesterpolyole, oder Polycarbonatpolyole, insbesondere aliphatische Polycarbonatpolyole.

Bevorzugt sind Polyole mit einem mittleren Molekulargewicht im Bereich von 400 bis 20'000 g/mol, bevorzugt von 1'000 bis 15'000 g/mol, besonders bevorzugt 1'500 bis 10'000 g/mol, insbesondere 2'000 bis 6'000 g/mol. Bevorzugt sind Polyole mit einer mittleren OH-Funktionalität im Bereich von 1.6 bis 4, insbesondere 1.6 bis 3.

Bei der Herstellung eines Isocyanatgruppen-haltigen Polyurethanpolymers können auch Anteile von zwei- oder mehrfunktionellen Alkoholen mitverwendet werden, insbesondere 1,2-Ethandiol, 1,2-Propandiol, 1,3-Propandiol, 2-Methyl-1,3-propandiol, 1,2-Butandiol, 1,3-Butandiol, 1,4-Butandiol, 1,3-Pentandiol, 1,5-Pentandiol, 3-Methyl-1,5-pentandiol, Neopentylglykol, Dibromneopentylglykol, 1,2-Hexandiol, 1,6-Hexandiol, 1,7-Heptandiol, 1,2-Octandiol, 1,8-Octandiol, 2-Ethyl-1,3-hexandiol, Diethylenglykol, Triethylenglykol, Dipropylenglykol, Tripropylenglykol, 1,3- oder 1,4-Cyclohexandimethanol, ethoxyliertes Bisphenol-A, propoxyliertes Bisphenol-A, Cyclohexandiol, hydriertes Bisphenol-A, Dimerfettsäurealkohole, 1,1,1-Trimethylolethan, 1,1,1-Trimethylolpropan, Glycerin, Pentaerythritol, Zuckeralkohole wie insbesondere Xylit, Sorbit oder Mannit oder Zucker wie insbesondere Saccharose oder alkoxylierte Derivate der genannten Alkohole oder Mischungen der genannten Alkohole.

Für die Herstellung von Umsetzungsprodukten mit Aldiminogruppen der Formel (V) bevorzugte Polyisocyanate sind HDI, IPDI, H₁₂MDI, TDI, MDI oder Oligomere oder Isocyanatgruppen-haltige Polyurethanpolymere dieser Isocyanate oder Mischungen davon.

Besonders geeignet sind bei Raumtemperatur flüssige oder bei Raumtemperatur feste Isocyanatgruppen-haltige Polyurethanpolymere, wobei deren Gehalt an monomeren Diisocyanaten bei der Umsetzung stark reduziert wird.

Ein bei Raumtemperatur festes Isocyanatgruppen-haltiges Polyurethanpolymer kann bei Raumtemperatur kristallin, teilkristallin oder amorph sein. Für ein teilkristallines oder amorphes Polyurethanpolymer gilt dabei, dass es bei Raumtemperatur nicht oder nur wenig fliessfähig ist. Das heisst insbesondere, dass es bei 20°C eine Viskosität von mehr als 5'000 Pa s aufweist.

Die Umsetzung eines Hydroxyaldimins der Formel (I) mit einem bei Raumtemperatur festen Isocyanatgruppen-haltigen Polyurethanpolymer erfolgt bevorzugt bei einer Temperatur, bei welcher das Polymer geschmolzen vorliegt, insbesondere bei einer Temperatur im Bereich von 80 bis 180°C.

Ebenfalls möglich ist die Herstellung eines Umsetzungsproduktes aus einem Hydroxyaldimin der Formel (I) und einem Isocyanatgruppen-haltigen Polyurethanpolymer, indem das Hydroxyaldimin der Formel (I) bereits bei der Herstellung des Isocyanatgruppen-haltigen Polyurethanpolymers zugegeben wird.

Das Umsetzungsprodukt mit Aldiminogruppen der Formel (V) weist insbesondere die Formel (VI) auf, 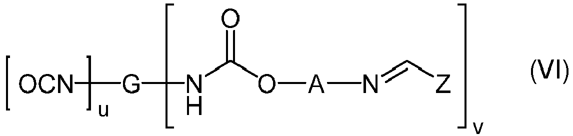 wobei
u für 0 oder eine ganze Zahl im Bereich von 1 bis 5 und v für eine ganze Zahl im Bereich von 1 bis 6 stehen, wobei (u+v) für eine ganze Zahl im Bereich von 2 bis 6 steht,
G für einen (u+v)-wertigen Kohlenwasserstoff-Rest mit einem mittleren Molekulargewicht im Bereich von 56 bis 20'000 g/mol, welcher gegebenenfalls Heteroatome aufweist, steht,
und A und Z die bereits genannten Bedeutungen aufweisen.

Bevorzugt steht u für 0 oder 1 oder 2 und v für 1 oder 2 oder 3 und (u+v) für 2 oder 3.

Bevorzugt steht G für den Rest eines der bevorzugten Polyisocyanate, insbesondere HDI, IPDI, H₁₂MDI, TDI, MDI oder Oligomere oder Isocyanatgruppen-haltige Polyurethanpolymere davon, nach Entfernung der Isocyanatgruppen.

In einem besonders bevorzugten Umsetzungsprodukt steht G für den Rest eines Isocyanatgruppen-haltigen Polyurethanpolymers nach Entfernung der Isocyanatgruppen und weist ein mittleres Molekulargewicht im Bereich von 500 bis 20'000 g/mol, insbesondere 1'000 bis 15'000 g/mol, bevorzugt 1'500 bis 10'000 g/mol, auf, und (u+v) steht im Mittel für einen Wert im Bereich von 1.8 bis 3, wobei der mittlere Wert von u grösser oder gleich dem mittleren Wert von v ist. Ein solches Umsetzungsprodukt weist im Mittel mindestens soviele Isocyanatgruppen wie Aldiminogruppen auf. Es wird insbesondere erhalten aus der Umsetzung von mindestens einem Hydroxyaldimin der Formel (V) mit mindestens einem Isocyanatgruppen-haltigen Polyurethanpolymer in einem OH/NCO-Verhältnis im Bereich von 0.05/1 bis 0.5/1, insbesondere 0.1/1 bis 0.5/1, bevorzugt 0.2/1 bis 0.5/1, wie vorgängig beschrieben.

Ein solches Umsetzungsprodukt kann auch Anteile von nicht-umgesetztem Isocyanatgruppen-haltigem Polyurethanpolymer enthalten. Bei einem solchen Umsetzungsprodukt steht v im Mittel insbesondere für einen Wert im Bereich von 0.1 bis 0.95.

In einem weiteren bevorzugten Umsetzungsprodukt steht G für den Rest eines Isocyanatgruppen-haltigen Polyurethanpolymers nach Entfernung der Isocyanatgruppen und weist ein mittleres Molekulargewicht im Bereich von 500 bis 20'000 g/mol, insbesondere 1'000 bis 15'000 g/mol, bevorzugt 1'500 bis 10'000 g/mol, auf, u steht für 0 und v steht im Mittel für einen Wert im Bereich von 1.8 bis 3. Ein solches Umsetzungsprodukt ist frei von Isocyanatgruppen. Es wird insbesondere erhalten aus der Umsetzung von mindestens einem Hydroxyaldimin der Formel (V) mit mindestens einem Isocyanatgruppen-haltigen Polyurethanpolymer in einem OH/NCO-Verhältnis im Bereich von 1/1 bis 1.5/1, insbesondere 1/1 bis 1.2/1, bevorzugt 1/1 bis 1.1/1, wie vorgängig beschrieben. Es weist die bereits genannten vorteilhaften Eigenschaften auf.

Die beschriebenen Umsetzungsprodukte sind besonders geeignet für die Verwendung in Isocyanatgruppen aufweisenden Zusammensetzungen, wobei sie entweder selber einen Teil oder alle der darin enthaltenen Isocyanatgruppen aufweisen oder mit weiteren Polyisocyanaten kombiniert werden können. Es sind sowohl einkomponentige als auch mehrkomponentige, insbesondere zweikomponentige, Zusammensetzungen möglich. Die Umsetzungsprodukte ermöglichen dabei die bereits genannten vorteilhaften Eigenschaften wie reduzierter Gehalt an monomeren Diisocyanaten, gute Lagerstabilität, kein Geruch, lange Offenzeit, gute mechanische Eigenschaften und kaum Neigung zu Weichmacher-Migration.

Ein weiterer Gegenstand der Erfindung ist eine Isocyanatgruppen-haltige Zusammensetzung enthaltend mindestens ein Umsetzungsprodukt mit Aldiminogruppen der Formel (V), wie vorgängig beschrieben.

In einer bevorzugten Ausführungsform enthält die Zusammensetzung ein Umsetzungsprodukt, welches selber mindestens soviele Isocyanatgruppen wie Aldiminogruppen aufweist. Dies ins insbesondere ein Umsetzungsprodukt, welches erhalten wurde aus der Umsetzung von mindestens einem Hydroxyaldimin der Formel (I) mit mindestens einem Isocyanatgruppen-haltigen Polyurethanpolymer im OH/NCO-Verhältnis im Bereich von 0.05/1 bis 0.5/1, insbesondere 0.1/1 bis 0.5/1, bevorzugt 0.2/1 bis 0.5/1. Eine solche Zusammensetzung weist den Vorteil auf, dass der Gehalt an monomeren Diisocyanaten besonders niedrig ist, da bei der Umsetzung mit dem Hydroxyaldimin ein überproportional grosser Anteil an monomerem Diisocyanat umgesetzt wurde.

Eine solche Zusammensetzung kann zusätzlich weitere Polyisocyanate enthalten. Bevorzugt enthält sie aber keine weiteren Polyisocyanate. Sie ist besonders geeignet als einkomponentige feuchtigkeitshärtende Zusammensetzung. Für den Fall, dass das Umsetzungsprodukt auf einem Isocyanatgruppen-haltigen Polyurethanpolymer beruht, enthält die Zusammensetzung einen besonders niedrigen Gehalt an monomeren Diisocyanaten. Insbesondere enthält sie einen Gehalt an monomeren Diisocyanaten unterhalb von 1 Gewichts-%, bevorzugt unterhalb von 0.5 Gewichts-%, insbesondere unterhalb von 0.3 Gewichts-%. Am meisten bevorzugt enthält sie weniger als 0.1 Gewichts-% monomere Diisocyanate.

In einer weiteren bevorzugten Ausführungsform enthält die Zusammensetzung ein Umsetzungsprodukt, welches selbst frei ist von Isocyanatgruppen. Bevorzugt ist das Isocyanatgruppen-freie Umsetzungsprodukt bei Raumtemperatur flüssig und weist ein mittleres Molekulargewicht im Bereich von 56 bis 20'000 g/mol, insbesondere 500 bis 10'000 g/mol, auf.

In diesem Fall enthält die Zusammensetzung zusätzlich mindestens ein Polyisocyanat, wobei dieselben Polyisocyanate geeignet sind wie die bereits genannten, insbesondere eine flüssige Form von MDI oder PMDI oder ein Oligomeres von HDI oder IPDI oder TDI oder ein Isocyanatgruppen-haltiges Polyurethanpolymer von HDI, IPDI, H₁₂MDI, TDI oder MDI, oder eine Mischung der genannten Polyisocyanate. Eine solche Zusammensetzung ist geeignet als einkomponentige oder als zweikomponentige Zusammensetzung.

Eine einkomponentige Zusammensetzung weist den Vorteil auf, dass sie einfacher verarbeitbar ist.

Im Fall einer zweikomponentigen Zusammensetzung können das Umsetzungsprodukt mit Aldiminogruppen der Formel (V) und das Polyisocyanat in der gleichen oder in getrennten Komponenten vorhanden sein. Bevorzugt liegen sie in getrennten Komponenten vor. Bevorzugt liegt dem Umsetzungsprodukt dabei ein Polyurethanpolymer zugrunde und das Polyisocyanat ist eine bei Raumtemperatur flüssige Form von MDI oder PMDI oder ein Oligomeres von HDI oder IPDI oder TDI.

Eine bei Raumtemperatur flüssige Form von MDI stellt entweder durch partielle chemische Modifizierung - insbesondere Carbodiimidisierung bzw. Uretoniminbildung oder Adduktbildung mit Polyolen - verflüssigtes 4,4'-MDI dar, oder es ist ein durch Abmischen gezielt herbeigeführtes oder durch den Herstellungsprozess bedingtes Gemisch von 4,4'-MDI mit anderen MDI-Isomeren (2,4'-MDI und/oder 2,2'-MDI), und/oder MDI-Oligomeren und/oder MDI-Homologen (PMDI).

Eine solche zweikomponentige Zusammensetzung weist den Vorteil auf, dass die auf dem Polyurethanpolymer beruhende Komponente nur Aldimino-funktionell und somit nicht empfindlich auf Kontakt mit Feuchtigkeit ist, solange sie nicht mit Isocyanatgruppen in Berührung kommt. Sie kann deshalb einfach mit weiteren Inhaltsstoffen formuliert und gefüllt werden. Es kann dabei vorteilhaft sein, wenn einer solchen Aldimino-funktionellen Komponente etwas Wasser zugesetzt wird, insbesondere in einer solchen Menge, dass die Komponente bis zu 2 Gewichts-%, insbesondere bis zu 1 Gewichts-%, Wasser enthält. Dadurch wird die Aushärtung beschleunigt.

Im Fall einer zweikomponentigen Zusammensetzung enthält die vom Polyisocyanat getrennte Komponente gegebenenfalls weitere mit Isocyanatgruppen reaktive Verbindungen, insbesondere zwei- oder mehrfunktionelle Alkohole oder Polyole oder Aminoalkohole oder Polyamine oder weitere latente Härter wie insbesondere Ketimine, Enamine, Oxazolidine oder nicht der Formel (I) entsprechende Aldimine.

Bevorzugt enthält die zweikomponentige Zusammensetzung ein Polyol, insbesondere mit einem mittleren Molekulargewicht im Bereich von 400 bis 10'000 g/mol, bevorzugt 500 bis 6'000 g/mol, mit einer mittleren OH-Funktionalität im Bereich von 1.6 bis 3, besonders bevorzugt 1.8 bis 3, und mit zumindest teilweise primären Hydroxylgruppen, also Hydroxylgruppen, welche an eine CH₂-Einheit gebunden sind, insbesondere ein Polyetherpolyol oder ein Polyhydroxy-funktionelles Fett oder Öl. In einem Polyetherpolyol können insbesondere auch darin dispergierten Polymerpartikeln, insbesondere Styrol-Acrylnitril-Partikel (SAN) oder Acrylnitril-Methylmethacrylat-Partikel, enthalten sein.

Bevorzugt enthält die zweikomponentige Zusammensetzung weiterhin mindestens einen zwei- oder mehrfunktionellen Alkohol, insbesondere 1,4-Butandiol.

Bevorzugt enthält die Isocyanatgruppen aufweisende Zusammensetzung zusätzlich einen oder mehrere weitere Bestandteile, welche insbesondere ausgewählt sind aus Katalysatoren, Füllstoffen, Weichmachern und Lösemitteln.

Im Fall einer zweikomponentigen Zusammensetzung können solche Bestandteile nur in einer oder in beiden Komponenten vorhanden sein.

Geeignete Katalysatoren sind insbesondere Katalysatoren für die Hydrolyse der Aldiminogruppen, insbesondere organische Säuren, insbesondere Carbonsäuren wie 2-Ethylhexansäure, Laurinsäure, Stearinsäure, Isostearinsäure, Ölsäure, Neodecansäure, Benzoesäure, Salicylsäure oder 2-Nitrobenzoesäure, organische Carbonsäureanhydride wie Phthalsäureanhydrid, Hexahydrophthalsäureanhydrid oder Hexahydromethylphthalsäureanhydrid, Silylester von Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester. Besonders bevorzugt sind Carbonsäuren, insbesondere aromatische Carbonsäuren wie Benzoesäure, 2-Nitrobenzoesäure oder insbesondere Salicylsäure.

Geeignete Katalysatoren sind weiterhin Katalysatoren für die Beschleunigung der Reaktion von Isocyanatgruppen, insbesondere Organozinn(IV)-Verbindungen wie insbesondere Dibutylzinndiacetat, Dibutylzinndilaurat, Dibutylzinndichlorid, Dibutylzinndiacetylacetonat, Dimethylzinndilaurat, Dioctylzinndiacetat, Dioctylzinndilaurat oder Dioctylzinndiacetylacetonat, Komplexverbindungen von Bismut(III) oder Zirkonium(IV), insbesondere mit Liganden ausgewählt aus Alkoholaten, Carboxylaten, 1,3-Diketonaten, Oxinat, 1,3-Ketoesteraten und 1,3-Ketoamidaten, oder tertiäre Aminogruppen enthaltende Verbindungen wie insbesondere 2,2'-Dimorpholinodiethylether (DMDEE).

Geeignet sind insbesondere auch Kombinationen von verschiedenen Katalysatoren.

Geeignete Füllstoffe sind insbesondere gemahlene oder gefällte Calciumcarbonate, welche gegebenenfalls mit Fettsäuren, insbesondere Stearaten, beschichtet sind, Baryte (Schwerspate), Quarzmehle, Quarzsande, Dolomite, Wollastonite, Kaoline, calcinierte Kaoline, Schichtsilikate wie Glimmer oder Talk, Zeolithe, Aluminiumhydroxide, Magnesiumhydroxide, Kieselsäuren inklusive hochdisperse Kieselsäuren aus Pyrolyseprozessen, Zemente, Gipse, Flugaschen, industriell hergestellte Russe, Graphit, Metall-Pulver, beispielsweise von Aluminium, Kupfer, Eisen, Silber oder Stahl, PVC-Pulver oder Hohlkugeln.

Geeignete Weichmacher sind insbesondere Carbonsäureester wie Phthalate, insbesondere Diisononylphthalat (DINP), Diisodecylphthalat (DIDP) oder Di(2-propylheptyl)phthalat (DPHP), hydrierte Phthalate, insbesondere hydriertes Diisononylphthalat (DINCH), Terephthalate, insbesondere Dioctylterephthalat, Trimellitate, Adipate, insbesondere Dioctyladipat, Azelate, Sebacate, Benzoate, Glykolether, Glykolester, organische Phosphor- oder Sulfonsäureester, Polybutene, Polyisobutene oder von natürlichen Fetten oder Ölen abgeleitete Weichmacher, insbesondere epoxidiertes Soja- oder Leinöl.

Geeignete Lösemittel sind insbesondere Aceton, Methylethylketon, Methyl-n-propylketon, Diisobutylketon, Methylisobutylketon, Methyl-n-amylketon, Methylisoamylketon, Acetylaceton, Mesityloxid, Cyclohexanon, Methylcyclohexanon, Ethylacetat, Propylacetat, Butylacetat, n-Butylpropionat, Diethylmalonat, 1-Methoxy-2-propylacetat, Ethyl-3-ethoxypropionat, Diisopropylether, Diethylether, Dibutylether, Diethylenglykoldiethylether, Ethylenglykoldiethylether, Ethylenglykolmonopropylether, Ethylenglykolmono-2-ethylhexylether, Toluol, Xylol, Heptan, Octan, Naphtha, White Spirit, Petrolether oder Benzin, insbesondere Solvesso^{™}-Typen (von Exxon), weiterhin Methylenchlorid, Propylencarbonat, Butyrolacton, N-Methylpyrrolidon oder N-Ethylpyrrolidon.

Die Zusammensetzung kann weitere für Polyurethanzusammensetzungen gebräuchliche Zusätze enthalten. Insbesondere können die folgenden Hilfs- und Zusatzstoffe vorhanden sein:
- nicht-reaktive thermoplastische Polymere, insbesondere Homo- oder Copolymere von ungesättigten Monomeren, insbesondere aus der Gruppe umfassend Ethylen, Propylen, Butylen, Isobutylen, Isopren, Vinylacetat und Alkyl(meth)acrylat, insbesondere Polyethylen (PE), Polypropylen (PP), Polyisobutylen, Ethylenvinylacetat-Copolymere (EVA) und ataktische Poly-α-Olefine (APAO); weiterhin Polyester, Polyacrylate, Polymethacrylate, Polyacrylamide, Polyacrylonitrile, Polyimide, Polyamide, Polyvinylchloride, Polysiloxane, Polyurethane, Polystyrole, und Kombinationen davon, insbesondere Polyetheramid Copolymere, Styrol-Butadien-Styrol Copolymere, Styrol-Isopren-Styrol Copolymere, Styrol-Ethylen-Butylen-Styrol Coplymere, Styrol-Ethylen-Propylen-Styrol Coplymere; sowie weiterhin Butylkautschuk, Polyisobutylen und Kombinationen davon, weiterhin Asphalt, Bitumen, Rohkautschuk, fluorierter Kautschuk oder Celluloseharze;
- Klebrigmacherharze, insbesondere ein Kohlenwasserstoffharz wie insbesondere Cumaron-Inden-Harze, Terpenharze, phenolmodifizierte Terpenharze, natürliche, gegebenenfalls modifizierte, Harze wie insbesondere Kolophonium, Wurzelharz oder Tallölharz, weiterhin α-Methyl-Styrolharze und polymere Milchsäure;
- natürliche Harze, Fette oder Öle wie Kolophonium, Schellack, Leinöl, Rizinusöl oder Sojaöl;
- anorganische oder organische Pigmente, insbesondere Titandioxid, Chromoxide oder Eisenoxide;
- Fasern, insbesondere Glasfasern, Kohlefasern, Metallfasern, Keramikfasern, Kunststofffasern wie Polyamidfasern oder Polyethylenfasern oder Naturfasern wie Wolle, Cellulose, Hanf oder Sisal;
- Farbstoffe;
- Trocknungsmittel, insbesondere Molekularsiebpulver, Calciumoxid, hochreaktive Isocyanate wie p-Tosylisocyanat, monomere Diisocyanate, Mono-Oxazolidine wie Incozol^{®} 2 (von Incorez) oder Orthoameisensäureester;
- Haftvermittler, insbesondere Organoalkoxysilane, insbesondere Epoxysilane wie insbesondere 3-Glycidoxypropyltrimethoxysilan oder 3-Glycidoxypropyltriethoxysilan, (Meth)acrylosilane, Anhydridosilane, Carbamatosilane, Alkylsilane oder Iminosilane, oder oligomere Formen dieser Silane, oder Titanate;
- latente Härter oder Vernetzer, insbesondere Aldimine, Ketimine, Enamine oder Oxazolidine;
- weitere Katalysatoren, welche die Reaktion der Isocyanatgruppen beschleunigen, insbesondere Salze, Seifen oder Komplexe von Zinn, Zink, Bismut, Eisen, Aluminium, Molybdän, Dioxomolybdän, Titan, Zirkonium oder Kalium, insbesondere Zinn(II)-2-ethylhexanoat, Zinn(II)-neodecanoat, Zink(II)-acetat, Zink(II)-2-ethylhexanoat, Zink(II)-laurat, Zink(II)-acetylacetonat, Aluminiumlactat, Aluminiumoleat, Diisopropoxytitan-bis-(ethylacetoacetat) oder Kaliumacetat; tertiäre Aminogruppen enthaltende Verbindungen, insbesondere N-Ethyldiisopropylamin, N,N,N',N'-Tetramethylalkylendiamine, Pentamethylalkylentriamine und höhere Homologe davon, Bis-(N,N-diethylaminoethyl)-adipat, Tris(3-dimethylaminopropyl)amin, 1,4-Diazabicyclo[2.2.2]octan (DABCO), 1,8-Diazabicyclo[5.4.0]undec-7-en (DBU), 1,5-Diazabicyclo[4.3.0]-non-5-en (DBN), N-Alkylmorpholine, N,N'-Dimethylpiperazin; stickstoffaromatische Verbindungen wie 4-Dimethylamino-pyridin, N-Methylimidazol, N-Vinylimidazol oder 1,2-Dimethylimidazol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen;
- Rheologie-Modifizierer, insbesondere Verdickungsmittel, insbesondere Schichtsilikate wie Bentonite, Derivate von Rizinusöl, hydriertes Rizinusöl, Polyamide, Polyamidwachse, Polyurethane, Harnstoffverbindungen, pyrogene Kieselsäuren, Celluloseether oder hydrophob modifizierte Polyoxyethylene;
- flammhemmende Substanzen, insbesondere die bereits genannten Füllstoffe Aluminiumhydroxid oder Magnesiumhydroxid, sowie insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis- oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- Additive, insbesondere Netzmittel, Verlaufmittel, Entschäumer, Entlüfter, Stabilisatoren gegen Oxidation, Wärme, Licht oder UV-Strahlung oder Biozide;
oder weitere üblicherweise in feuchtigkeitshärtenden Zusammensetzungen eingesetzte Substanzen.

Es kann sinnvoll sein, gewisse Substanzen vor dem Einmischen in die Zusammensetzung chemisch oder physikalisch zu trocknen.

Die Herstellung des in der Zusammensetzung enthaltenen Umsetzungsprodukts mit Aldiminogruppen der Formel (V) kann auch in-situ in einer ansonsten voll formulierten Isocyanatgruppen aufweisenden Zusammensetzung erfolgen, indem dieser unter Ausschluss von Feuchtigkeit mindestens ein Hydroxyaldimin der Formel (I) zugemischt wird und die Hydroxylgruppen mit vorhandenen Isocyanatgruppen reagieren.

In der Zusammensetzung liegt das Verhältnis zwischen den gegenüber Isocyanaten reaktiven Gruppen inklusive Aldiminogruppen und den Isocyanatgruppen bevorzugt im Bereich von 0.2 bis 1.2, besonders bevorzugt im Bereich von 0.4 bis 1.0, insbesondere im Bereich von 0.5 bis 0.95.

Bevorzugt liegt das Verhältnis zwischen Aldiminogruppen und Isocyanatgruppen im Bereich von 0.05 bis 1.1, besonders bevorzugt im Bereich von 0.1 bis 1.0, insbesondere im Bereich von 0.2 bis 0.9.

Die Zusammensetzung wird insbesondere unter Ausschluss von Feuchtigkeit hergestellt, wobei die Bestandteile der Zusammensetzung zu einer makroskopisch homogenen Masse vermischt werden, und bei Umgebungstemperatur in feuchtigkeitsdichten Gebinden aufbewahrt. Ein geeignetes feuchtigkeitsdichtes Gebinde besteht insbesondere aus einem gegebenenfalls beschichteten Metall und/oder Kunststoff und stellt insbesondere ein Fass, ein Container, ein Hobbock, ein Eimer, ein Kanister, eine Büchse, ein Beutel, ein Schlauchbeutel, eine Kartusche oder eine Tube dar.

Die Zusammensetzung kann in Form einer einkomponentigen oder einer mehrkomponentigen, insbesondere zweikomponentigen, Zusammensetzung vorliegen.

Bei der Applikation der Zusammensetzung beginnt der Prozess der Vernetzung. Als Ergebnis davon entsteht die ausgehärtete vernetzte Zusammensetzung.

Eine einkomponentige Zusammensetzung wird als solche appliziert und beginnt darauf unter dem Einfluss von Feuchtigkeit bzw. Wasser auszuhärten bzw. zu vernetzen. Zur Beschleunigung der Vernetzung kann der Zusammensetzung bei der Applikation eine Beschleuniger-Komponente, welche Wasser und/oder einen Katalysator enthält oder freisetzt, zugemischt werden, oder die Zusammensetzung kann nach ihrer Applikation mit einer solchen Beschleuniger-Komponente in Kontakt gebracht werden.

Eine zweikomponentige Zusammensetzung wird nach dem Mischen der beiden Komponenten appliziert und beginnt dabei durch interne Reaktion zu vernetzen, wobei die Vernetzung gegebenenfalls durch die Einwirkung von externer Feuchtigkeit vervollständigt wird.

Bei der Aushärtung bzw. Vernetzung reagieren die Isocyanatgruppen unter dem Einfluss von Feuchtigkeit mit den Aldiminogruppen und gegebenenfalls vorhandenen weiteren blockierten Aminogruppen. Ein Teil der Isocyanatgruppen, insbesondere die gegenüber den Aldiminogruppen überschüssigen, reagieren unter dem Einfluss von Feuchtigkeit untereinander und/oder mit gegebenenfalls in der Zusammensetzung vorhandenen weiteren Reaktivgruppen, insbesondere Hydroxylgruppen oder freien Aminogruppen. Die Gesamtheit dieser zur Aushärtung der Zusammensetzung führenden Reaktionen der Isocyanatgruppen wird auch als Vernetzung bezeichnet.

Die zur Aushärtung einer einkomponentigen Zusammensetzung benötigte Feuchtigkeit gelangt bevorzugt aus der Luft (Luftfeuchtigkeit) durch Diffusion in die Zusammensetzung. Dabei bildet sich an den mit Luft in Kontakt stehenden Oberflächen der Zusammensetzung eine feste Schicht ausgehärteter Zusammensetzung ("Haut"). Die Aushärtung setzt sich entlang der Diffusionsrichtung von aussen nach innen fort, wobei die Haut zunehmend dicker wird und schliesslich die ganze applizierte Zusammensetzung umfasst. Die Feuchtigkeit kann zusätzlich oder vollständig auch aus einem oder mehreren Substrat(en), auf welche(s) die Zusammensetzung appliziert wurde, in die Zusammensetzung gelangen und/oder aus einer Beschleuniger-Komponente stammen, welche der Zusammensetzung bei der Applikation zugemischt oder nach der Applikation mit dieser in Kontakt gebracht wird, beispielsweise durch Bestreichen oder Besprühen.

Die zur Vervollständigung der Aushärtung einer zweikomponentigen Zusammensetzung gegebenenfalls benötigte externe Feuchtigkeit stammt bevorzugt aus der Luft und/oder aus den Substraten.

Die Zusammensetzung verfügt über eine vergleichsweise lange Offenzeit.

Als "Offenzeit" wird die Zeitspanne bezeichnet, während der die Zusammensetzung verarbeitet oder nachbearbeitet werden kann, nachdem der Aushärtungsprozess begonnen hat.

Die Zeit bis zur Ausbildung einer Haut ("Hautbildungszeit" oder "tack-free time") stellt dabei ein Mass für die Offenzeit dar.

Bei der Vernetzung wird ein Aldehyd der Formel (IV) freigesetzt. Dieser ist weitgehend unflüchtig und geruchlos und verbleibt zum grössten Teil in der ausgehärteten Zusammensetzung. Dort verhält er sich bzw. wirkt wie ein Weichmacher. Als solcher kann er grundsätzlich selber migrieren und/oder die Migration von Weichmachern beeinflussen. Der Aldehyd der Formel (IV) ist mit der ausgehärteten Zusammensetzung sehr gut verträglich, migriert selber kaum und löst auch keine verstärkte Migration von Weichmachern aus.

Bevorzugt stellt die Zusammensetzung einen Klebstoff oder einen Dichtstoff oder eine Beschichtung dar.

In einer bevorzugten Ausführungsform ist der Klebstoff oder Dichtstoff oder die Beschichtung elastisch und wird typischerweise bei Umgebungstemperatur appliziert, insbesondere im Bereich von etwa 0 bis 50°C, bevorzugt im Bereich von 5 bis 40°C.

Ein solcher Klebstoff und/oder Dichtstoff kann eine pastöse Konsistenz mit strukturviskosen Eigenschaften aufweisen und mittels einer geeigneten Vorrichtung, beispielsweise aus handelsüblichen Kartuschen oder Fässern oder Hobbocks, appliziert werden, beispielsweise in Form einer Raupe, wobei diese eine im Wesentlichen runde oder dreieckige Querschnittsfläche aufweisen kann. Er kann aber auch flüssig oder nur leicht thixotrop und selbstverlaufend sein.

Er ist geeignet für Klebe- und Abdichtungsanwendungen, insbesondere in der Bau- und Fertigungsindustrie oder im Fahrzeugbau, insbesondere für die Parkettverklebung, Anbauteilverklebung, Hohlraumversiegelung, Montage, Modulverklebung, Karosserieverklebung, Scheibenverklebung, Fugenabdichtung oder Verankerung.

Elastische Verklebungen im Fahrzeugbau sind beispielsweise das Ankleben von Teilen wie Kunststoffabdeckungen, Zierleisten, Flansche, Stosstangen, Führerkabinen oder andere Anbauteile, an die lackierte Karosserie eines Fahrzeugs, oder das Einkleben von Scheiben in die Karosserie, wobei die Fahrzeuge insbesondere Automobile, Lastkraftwagen, Busse, Schienenfahrzeuge oder Schiffe darstellen.

Als Dichtstoff ist die Zusammensetzung insbesondere geeignet für das elastische Abdichten von Fugen, Nähten oder Hohlräumen aller Art, insbesondere von Fugen am Bau wie Dilatationsfugen oder Anschlussfugen zwischen Bauteilen.

Eine elastische Beschichtung kann eine flüssige Konsistenz aufweisen und selbstverlaufend auf vorwiegend ebene Flächen appliziert werden, oder sie kann eine gewisse Strukturviskosität aufweisen, so dass sie bei der Applikation auf geneigte Flächen nicht abfliesst.

Als Beschichtung ist die Zusammensetzung insbesondere geeignet zum Schutz von Böden oder Mauern, insbesondere für Balkone, Terrassen, Plätze, Brücken, Parkdecks, oder zur Abdichtung von Dächern, insbesondere Flachdächer oder schwach geneigte Dachflächen oder Dachgärten, oder im Innern von Gebäuden zur Wasserabdichtung, beispielsweise unter Fliesen oder Keramikplatten in Nasszellen oder Küchen, oder als Bodenbelag in Küchen, Industriehallen oder Fabrikationsräumen, oder als Abdichtung in Auffangwannen, Kanälen, Schächten oder Abwasserbehandlungsanlagen, oder zum Schutz von Oberflächen als Lack oder Versiegelung, oder als Vergussmasse zur Hohlraumversiegelung, als Nahtabdichtung oder als Schutzbeschichtung für beispielsweise Rohre. Sie kann auch zu Reparaturzwecken als Abdichtung oder Beschichtung verwendet werden, beispielsweise von undichten Dachmembranen oder nicht mehr tauglichen Bodenbelägen oder insbesondere als Reparaturmasse für hochreaktive Spritzabdichtungen.

In einer besonders bevorzugten Ausführungsform stellt die Zusammensetzung einen reaktiven Heissschmelzklebstoff (PUR-RHM) dar. In diesem Fall ist die Zusammensetzung insbesondere einkomponentig und enthält insbesondere mindestens ein bei Raumtemperatur festes Isocyanatgruppen-haltiges Polyurethanpolymer, welches mit mindestens einem Hydroxyaldimin der Formel (I) in einem OH/NCO-Verhältnis im Bereich von 0.05/1 bis 0.5/1, insbesondere 0.1/1 bis 0.5/1, bevorzugt 0.2/1 bis 0.5/1, zu einem bei Raumtemperatur festen Umsetzungsprodukt mit Aldiminogruppen der Formel (V) umgesetzt ist.

Ein solcher reaktiver Heissschmelzklebstoff enthält einen besonders niedrigen Gehalt an monomeren Diisocyanaten. Dies ist aus toxikologischen Gründen besonders vorteilhaft, da bei der Verarbeitung im heissen Zustand die monomeren Diisocyanate vermehrt in die Umgebung gelangen und den Verarbeiter somit besonders stark belasten.

Der reaktive Heissschmelzklebstoff weist bevorzugt einen Gehalt an bei Raumtemperatur festem Polyurethanpolymer inklusive Umsetzungsprodukt im Bereich von 5 bis 100 Gewichts-%, insbesondere 15 bis 95 Gewichts-%, besonders bevorzugt 30 bis 90 Gewichts-%, am meisten bevorzugt 50 bis 80 Gewichts-%, auf.

Bevorzugt enthält er mindestens ein weiteres Polymer ausgewählt aus der Gruppe bestehend aus nicht-reaktiven thermoplastischen Polymeren und Klebrigmacherharzen.

Bevorzugt weist er einen Gehalt an Polymeren inklusive dem bei Raumtemperatur festen Polyurethanpolymer inklusive Umsetzungsprodukt im Bereich von 70 bis 100 Gewichts-%, besonders bevorzugt 80 bis 100 Gewichts-%, insbesondere 90 bis 100 Gewichts-%, auf.

Der reaktive Heissschmelzklebstoff wird für die Applikation aufgeschmolzen und im geschmolzenen Zustand verarbeitet und appliziert. Die Verarbeitung erfolgt typischerweise bei einer Temperatur im Bereich von 80 bis 180°C, insbesondere 100 bis 180°C. Dabei soll der geschmolzene unvernetzte Klebstoff für einige Zeit, insbesondere während einigen Stunden, auf einer rotierenden Walze als niedrigviskose Flüssigkeit in Kontakt mit der Umgebungsfeuchte stabil bleiben, also insbesondere keine starke Viskositätserhöhung und keine Anhärtungen oder Ausfällungen zeigen. Dies gelingt mit dem erfindungsgemässen Heissschmelzklebstoff überraschend gut. Nach der Applikation erstarrt der Klebstoff beim Abkühlen sehr schnell, wodurch er quasi sofort aushärtet und Haftung aufbaut, aber im unvernetzten Zustand noch thermoplastisch ist und durch erneutes Erwärmen wieder verformt oder verflüssigt werden kann. Durch den Kontakt mit Feuchtigkeit vernetzen die vorhandenen Aldimino- und Isocyanatgruppen wie vorgängig beschrieben, wodurch der Klebstoff zusätzlich chemisch vernetzt. Somit wird ein ausgehärteter vernetzter Klebstoff erhalten, welcher durch erneutes Erhitzen auf die Applikationstemperatur nicht wieder aufschmelzbar ist.

Als reaktiver Heissschmelzklebstoff ist die Zusammensetzung insbesondere geeignet für Bau- und Industrieanwendungen, insbesondere als Kaschierklebstoff, Laminatklebstoff, Verpackungsklebstoff, Textilklebstoff oder Holzklebstoff. Besonders geeignet ist er für Verklebungen, in welchen die Verklebungsstelle sichtbar ist, insbesondere für das Verkleben von Glas, beispielsweise im Fahrzeug- und Fensterbau, oder für das Verkleben von Klarsichtverpackungen.

Geeignete Substrate, welche mit der Zusammensetzung verklebt oder abgedichtet oder beschichtet werden können, sind insbesondere
- Glas, Glaskeramik, Beton, Mörtel, Faserzement, insbesondere Faserzement-Platten, Backstein, Ziegel, Gips, insbesondere Gips-Platten, oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Kupfer, Eisen, Stahl, Buntmetalle, inklusive oberflächenveredelte Metalle oder Legierungen wie verzinkte oder verchromte Metalle;
- Asphalt oder Bitumen;
- Leder, Textilien, Papier, Holz, mit Harzen wie Phenol-, Melamin- oder Epoxidharzen gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- Isolierschäume, insbesondere aus EPS, XPS, PUR, PIR, Steinwolle, Glaswolle oder geschäumtem Glas (Foamglas);
- beschichtete oder lackierte Substrate, insbesondere lackierte Fliesen, gestrichener Beton, pulverbeschichtete Metalle oder Legierungen oder lackierte Bleche;
- Farben oder Lacke, insbesondere Automobildecklacke.

Die Substrate können bei Bedarf vor dem Applizieren vorbehandelt werden, insbesondere durch physikalische und/oder chemische Reinigungsverfahren oder das Aufbringen eines Aktivators oder eines Primers.

Verklebt und/oder abgedichtet werden können zwei gleichartige oder zwei verschiedene Substrate.

Für Verklebungen mit dem reaktiven Heissschmelzklebstoff bevorzugt sind Kunststoffe, Textilien, Leder, Holz, Holzwerkstoffe, Polymer-Composites, Papier, Metalle, Farben oder Lacke, wobei insbesondere zwei verschiedene Substrate verklebt werden.

Aus der Applikation und Aushärtung der Zusammensetzung wird ein Artikel erhalten, welcher mit der Zusammensetzung verklebt oder abgedichtet oder beschichtet ist. Dieser Artikel kann ein Bauwerk oder ein Teil davon sein, insbesondere ein Bauwerk des Hoch- oder Tiefbaus, eine Brücke, ein Dach, ein Treppenhaus oder eine Fassade, oder er kann ein industrielles Gut oder ein Konsumgut sein, insbesondere ein Holzfaserwerkstoff aus dem Duschen- und Badbereich, Möbel-Dekorfolien, Klarsichtverpackungen, Membranfolien mit Textilien wie insbesondere Baumwolle, Polyesterfolien im Bekleidungsbereich, Verbunde aus Textilien und Schäumen für Autoausstattungen, Fenster, Rohre, Rotorblätter von Windkraftanlagen, Haushaltmaschinen oder ein Transportmittel wie insbesondere ein Automobil, ein Bus, ein Lastkraftwagen, ein Schienenfahrzeug, ein Schiff, ein Flugzeug oder ein Helikopter, oder ein Anbauteil davon, insbesondere ein Automobil-Innenausstattungsteil wie insbesondere Dachhimmel, Sonnenblende, Instrumententafel, Türseitenteil oder Hutablage.

Die beschriebene Zusammensetzung zeichnet sich dadurch aus, dass sie besonders lagerstabil ist und gegebenenfalls einen besonders niedrigen Gehalt an monomeren Diisocyanaten aufweist.

Bei der Applikation als Klebstoff und/oder Dichtstoff oder Beschichtung weist sie eine vergleichsweise lange Offenzeit auf, wodurch über einen längeren Zeitraum nach der Applikation einen nahtlosen Verlauf des applizierten Materials oder eine Positionierung oder Nachjustierung der damit verbundenen Objekte ermöglicht wird, was beispielsweise bei grossflächigen Beschichtungen, langen Dichtstreifen oder bei der Verklebung von grossen oder komplexen Objekten entscheidend ist.

Bei der Applikation als reaktiver Heissschmelzklebstoff weist die Zusammensetzung im geschmolzenen Zustand in Kontakt mit der Umgebungsluft eine besonders gute Stabilität auf, wodurch der Klebstoff im heissen Zustand einige Stunden auf einer offenen Walze rotiert werden kann, ohne dass eine nennenswerte Viskositätserhöhung auftritt.

Die Vernetzung der Zusammensetzung verläuft blasenfrei, vollständig und ohne Geruchsimmissionen. Im ausgehärteten Zustand verfügt die Zusammensetzung über gute Festigkeiten und Dehnbarkeiten und neigt nicht zu Problemen mit Weichmacher-Migration.

Ein weiterer Gegenstand der Erfindung ist ein Verfahren zur Reduktion des Gehaltes an monomeren Diisocyanaten von einem Isocyanatgruppen-haltigen Polyurethanpolymer, indem dieses in einem unterstöchiometrischen OH/NCO-Verhältnis unter Ausschluss von Feuchtigkeit mit mindestens einem Hydroxyaldimin der Formel (I) umgesetzt wird, gegebenenfalls in Anwesenheit von Katalysatoren, Weichmachern, Füllstoffen und/oder Lösemitteln.

Dabei liegt das OH/NCO-Verhältnis bevorzugt im Bereich von 0.05/1 bis 0.5/1, insbesondere 0.1/1 bis 0.5/1, bevorzugt 0.2/1 bis 0.5/1.

Dabei ist das Isocyanatgruppen-haltige Polyurethanpolymer insbesondere bei Raumtemperatur fest und geeignet als Bestandteil eines reaktiven Heissschmelzklebstoffs.

Die Produkte aus diesem Verfahren verfügen zusätzlich zum geringen Gehalt an monomeren Diisocyanaten über eine geruchsfreie Aushärtung, gute Lagerstabilität, lange Offenzeit bei schneller Aushärtung und neigen nicht zu Problemen mit Weichmacher-Migration wie Ausschwitzen (Bleeding), Substratverfärbung oder Spannungsrissbildung im Substrat.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

### verwendete Aldehyde:

| | |
|---|---|
| Aldehyd-1: | Fraktioniertes Reaktionsgemisch erhalten aus mittels HF-BF₃ katalysierter Formylierung von C₁₀₋₁₄-Alkylbenzol, enthaltend mehrheitlich verzweigte 4-(C₁₀₋₁₄-Alkyl)benzaldehyde. (mittleres Aldehyd-Equivalentgewicht 290 g/Eq) |

2,2-Dimethyl-3-lauroyloxypropanal

Aldehyd-1 ist ein Gemisch von Aldehyden der Formel (IV). 2,2-Dimethyl-3-lau-royloxypropanal dient als Vergleich.

### Herstellung von Hydroxyaldiminen:

Die **Aminzahl** (inklusive Aldiminogruppen) wurde bestimmt mittels Titration (mit 0.1N HClO₄ in Essigsäure gegen Kristallviolett).

Die **Viskosität** wurde mit einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.

### Hydroxyaldimin A1:

25.00 g Aldehyd-1 wurden in einem Rundkolben unter Stickstoffatmosphäre vorgelegt. Unter Rühren wurden 8.60 g 2-(2-Aminoethoxy)ethanol zugegeben und anschliessend die flüchtigen Bestandteile bei 80 °C und 10 mbar Vakuum entfernt. Es wurde eine blassgelbe, geruchlose und pH-neutrale Flüssigkeit mit einer Viskosität bei 20°C von 0.4 Pa·s und einer Aminzahl von 142.9 mg KOH/g erhalten.

### Hydroxyaldimin A2:

25.00 g Aldehyd-1 wurden in einem Rundkolben unter Stickstoffatmosphäre vorgelegt. Unter Rühren wurden 14.02 g 3-Aminomethyl-3,5,5-trimethylcyclo-hexanol zugegeben und anschliessend die flüchtigen Bestandteile bei 80°C und 10 mbar Vakuum entfernt. Es wurde eine blassgelbe, geruchlose und pH-neutrale Flüssigkeit mit einer Viskosität bei 20°C von 34.3 Pa·s und einer Aminzahl von 122.3 mg KOH/g erhalten.

### Hydroxyaldimin R1:

24.46 g 2,2-Dimethyl-3-lauroyloxypropanal wurden in einem Rundkolben unter Stickstoffatmosphäre vorgelegt. Unter Rühren wurden 8.60 g 2-(2-Aminoethoxy)ethanol zugegeben und anschliessend die flüchtigen Bestandteile bei 80°C und 10 mbar Vakuum entfernt. Es wurde eine fast farblose, geruchlose Flüssigkeit mit einer Aminzahl von 144.3 mg KOH/g erhalten.

### Hydroxyaldimin R2:

24.46 g 2,2-Dimethyl-3-lauroyloxypropanal wurden in einem Rundkolben unter Stickstoffatmosphäre vorgelegt. Unter Rühren wurden 14.02 g 3-Aminomethyl-3,5,5-trimethylcyclohexanol zugegeben und anschliessend die flüchtigen Bestandteile bei 80°C und 10 mbar Vakuum entfernt. Es wurde eine blassgelbe, geruchlose Flüssigkeit mit einer Aminzahl von 124.2 mg KOH/g erhalten.

Die Hydroxyaldimine **A1** und **A2** stellen Hydroxyaldimine der Formel (I) dar und sind erfindungsgemässe Beispiele. Die Hydroxyaldimine **R1** und **R2** sind Vergleichsbeispiele.

### Herstellung von Isocyanatgruppen-haltigen Polymeren

### Polymer P1:

4000 g Polyoxypropylen-Diol (Acclaim^{®} 4200, von Covestro; OH-Zahl 28.5 mg KOH/g) und 520 g 4,4'-Diphenylmethandiisocyanat (Desmodur^{®} 44 MC L, von Covestro) wurden nach bekanntem Verfahren bei 80 °C zu einem bei Raumtemperatur flüssigen, NCO-terminierten Polyurethanpolymer mit einem Gehalt an freien Isocyanatgruppen von 1.85 Gewichts-% umgesetzt.

### Polymer P2:

300.0 g Polyoxypropylenpolyoxyethylen-Diol (Desmophen^{®} L300, von Covestro; OH-Zahl 190.0 mg KOH/g) und 228.8 g Isophorondiisocyanat (Vestanat^{®} IPDI, Degussa) wurden nach bekanntem Verfahren bei 60°C zu einem bei Raumtemperatur flüssigen, NCO-terminierten Polyurethanpolymer mit einem Gehalt an freien Isocyanatgruppen von 8.35 Gewichts-% umgesetzt.

### Herstellung von Umsetzungsprodukten der Hydroxyaldimine:

### Umsetzungsprodukt U1:

227.30 g (ca. 100 mmol NCO) Polymer P1, 9.41 g (ca. 24 mmol) Hydroxyaldimin A1 und 0.13 g Coscat^{®} 83 (Bi(III)-neodecanoat, von Vertellus) wurden in einem Planetenmischer unter Vakuum bei 70°C während 90 Minuten gerührt. Es wurde ein klares, bei Raumtemperatur flüssiges, Aldimino- und Isocyanatgruppen aufweisendes Umsetzungsprodukt erhalten.

### Umsetzungsprodukt U2:

227.30 g (ca. 100 mmol NCO) Polymer P1, 11.02 g (ca. 24 mmol) Hydroxyaldimin A2 und 0.13 g Coscat^{®} 83 wurden in einem Planetenmischer unter Vakuum bei 70°C während 90 Minuten gerührt. Es wurde ein klares, bei Raumtemperatur flüssiges, Aldimino- und Isocyanatgruppen aufweisendes Umsetzungsprodukt erhalten.

### Umsetzungsprodukt U3:

227.30 g (ca. 100 mmol NCO) Polymer P1, 18.82 g (ca. 48 mmol) Hydroxyaldimin A1 und 0.13 g Coscat^{®} 83 wurden in einem Planetenmischer unter Vakuum bei 70°C während 90 Minuten gerührt. Es wurde ein klares, bei Raumtemperatur flüssiges, Aldimino- und Isocyanatgruppen aufweisendes Umsetzungsprodukt erhalten.

### Umsetzungsprodukt U4:

227.30 g (ca. 100 mmol NCO) Polymer P1, 22.05 g (ca. 48 mmol) Hydroxyaldimin A2 und 0.13 g Coscat^{®} 83 wurden in einem Planetenmischer unter Vakuum bei 70°C während 90 Minuten gerührt. Es wurde ein klares, bei Raumtemperatur flüssiges, Aldimino- und Isocyanatgruppen aufweisendes Umsetzungsprodukt erhalten.

### Umsetzungsprodukt U5:

227.30 g (ca. 100 mmol NCO) Polymer P1, 39.61 g (ca. 101 mmol) Hydroxyaldimin A1 und 0.13 g Coscat^{®} 83 wurden in einem Planetenmischer unter Vakuum bei 70°C während 90 Minuten gerührt. Es wurde ein klares, bei Raumtemperatur flüssiges, Aldiminogruppen aufweisendes Umsetzungsprodukt erhalten, welches frei von Isocyanatgruppen war.

### Umsetzungsprodukt U6:

227.30 g (ca. 100 mmol NCO) Polymer P1, 46.42 g (ca. 101 mmol) Hydroxyaldimin A2 und 0.13 g Coscat^{®} 83 wurden in einem Planetenmischer unter Vakuum bei 70°C während 90 Minuten gerührt. Es wurde ein klares, bei Raumtemperatur flüssiges, Aldiminogruppen aufweisendes Umsetzungsprodukt erhalten, welches frei von Isocyanatgruppen war.

### Umsetzungsprodukt U7:

60.00 g (ca. 115 mmol NCO) Polymer P3, 15.82 g (ca. 40.4 mmol) Hydroxyaldimin A1 und 0.05 g Coscat^{®} 83 wurden in einem Honigglas unter Argon vermischt und anschliessend während 16 Stunden in einem Umluftofen bei 60°C reagieren lassen. Es wurde ein klares, bei Raumtemperatur flüssiges, Aldimino- und Isocyanatgruppen aufweisendes Umsetzungsprodukt erhalten.

### Umsetzungsprodukt U8:

60.00 g (ca. 115 mmol NCO) Polymer P3, 18.51 g (ca. 40.4 mmol) Hydroxyaldimin A2 und 0.05 g Coscat^{®} 83 wurden in einem Honigglas unter Argon vermischt und anschliessend während 16 Stunden in einem Umluftofen bei 60°C reagieren lassen. Es wurde ein klares, bei Raumtemperatur flüssiges, Aldimino- und Isocyanatgruppen aufweisendes Umsetzungsprodukt erhalten.

### Umsetzungsprodukt Q1:

227.30 g (ca. 100 mmol NCO) Polymer P1, 9.33 g (ca. 24 mmol) Hydroxyaldimin R1 und 0.13 g Coscat^{®} 83 wurden in einem Planetenmischer unter Vakuum bei 70°C während 90 Minuten gerührt. Es wurde ein klares, bei Raumtemperatur flüssiges, Aldimino- und Isocyanatgruppen aufweisendes Umsetzungsprodukt erhalten.

### Umsetzungsprodukt Q2:

227.30 g (ca. 100 mmol NCO) Polymer P1, 10.84 g (ca. 24 mmol) Hydroxyaldimin R2 und 0.13 g Coscat^{®} 83 wurden in einem Planetenmischer unter Vakuum bei 70°C während 90 Minuten gerührt. Es wurde ein klares, bei Raumtemperatur flüssiges, Aldimino- und Isocyanatgruppen aufweisendes Umsetzungsprodukt erhalten.

### Umsetzungsprodukt Q3:

227.30 g (ca. 100 mmol NCO) Polymer P1, 18.66 g (ca. 48 mmol) Hydroxyaldimin R1 und 0.13 g Coscat^{®} 83 wurden in einem Planetenmischer unter Vakuum bei 70°C während 90 Minuten gerührt. Es wurde ein klares, bei Raumtemperatur flüssiges, Aldimino- und Isocyanatgruppen aufweisendes Umsetzungsprodukt erhalten.

### Umsetzungsprodukt Q4:

227.30 g (ca. 100 mmol NCO) Polymer P1, 21.68 g (ca. 48 mmol) Hydroxyaldimin R2 und 0.13 g Coscat^{®} 83 wurden in einem Planetenmischer unter Vakuum bei 70°C während 90 Minuten gerührt. Es wurde ein klares, bei Raumtemperatur flüssiges, Aldimino- und Isocyanatgruppen aufweisendes Umsetzungsprodukt erhalten.

### Umsetzungsprodukt Q5:

227.30 g (ca. 100 mmol NCO) Polymer P1, 39.26 g (ca. 101 mmol) Hydroxyaldimin R1 und 0.13 g Coscat^{®} 83 wurden in einem Planetenmischer unter Vakuum bei 70°C während 90 Minuten gerührt. Es wurde ein klares, bei Raumtemperatur flüssiges, Aldiminogruppen aufweisendes Umsetzungsprodukt erhalten, welches frei von Isocyanatgruppen war.

### Umsetzungsprodukt Q6:

227.30 g (ca. 100 mmol NCO) Polymer P1, 45.62 g (ca. 101 mmol) Hydroxyaldimin R2 und 0.13 g Coscat^{®} 83 wurden in einem Planetenmischer unter Vakuum bei 70°C während 90 Minuten gerührt. Es wurde ein klares, bei Raumtemperatur flüssiges, Aldiminogruppen aufweisendes Umsetzungsprodukt erhalten, welches frei von Isocyanatgruppen war.

### Umsetzungsprodukt Q7:

60.00 g (ca. 115 mmol NCO) Polymer P3, 15.69 g (ca. 40.4 mmol) Hydroxyaldimin R1 und 0.05 g Coscat^{®} 83 wurden in einem Honigglas unter Argon vermischt und anschliessend während 16 Stunden in einem Umluftofen bei 60°C reagieren lassen. Es wurde ein klares, bei Raumtemperatur flüssiges, Aldimino- und Isocyanatgruppen aufweisendes Umsetzungsprodukt erhalten.

Die Umsetzungsprodukte **U1** bis **U8** stellen Umsetzungsprodukte mit Aldiminogruppen der Formel (V) dar und sind erfindungsgemässe Beispiele. Die Umsetzungsprodukte **Q1** bis **Q7** sind Vergleichsbeispiele.

### Gehalt an monomeren Diisocyanaten

Von Polymer P1 und von einigen Umsetzungsprodukten davon wurde 14 Tage nach der Herstellung der Gehalt an monomerem 4,4'-MDI mittels HPLC (Detektion über Photodiodenarray; 0.04 M Natriumacetat / Acetonitril als mobile Phase) gemessen. Die Resultate sind in der Tabelle 1 angegeben.

**Tabelle 1**

| | **Gehalt an 4,4'-MDI** |
|---|---|
| Polymer **P1** | 2.52 Gew.-% |
| Umsetzungsprodukt **U1** | 0.99 Gew.-% |
| Umsetzungsprodukt **U3** | 0.35 Gew.-% |
| Umsetzungsprodukt **Q1** | 1.88 Gew.-% |
| Umsetzungsprodukt **Q3** | 0.20 Gew.-% |

### Einkomponentige Zusammensetzungen

### Zusammensetzungen Z1 bis Z3 und Ref1 bis Ref3

Für jede Zusammensetzung wurden die in der Tabelle 2 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt und unter Feuchtigkeitsausschluss aufbewahrt. Jede Zusammensetzung wurde folgendermassen geprüft:
Als Mass für die Lagerstabilität wurden die **Viskosität (1d RT)** am Folgetag der Herstellung und die **Viskosität (7d 60°C)** nach einer Lagerung im verschlossenen Gebinde während 7 Tagen in einem 60°C Umluftofen bestimmt. Die Viskosität wurde jeweils bei einer Temperatur von 20°C mit einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.

Als Mass für die Offenzeit wurde die Hautbildungszeit **(tack-free time)** bestimmt. Dazu wurden einige Gramm der Zusammensetzung in einer Schichtdicke von ca. 2 mm auf Pappkarton aufgetragen und im Normklima die Zeitdauer bestimmt, bis beim leichten Antippen der Oberfläche der Zusammensetzung mittels einer Pipette aus LDPE erstmals keine Rückstände auf der Pipette mehr zurückblieben.

Zur Bestimmung der mechanischen Eigenschaften wurde jede Zusammensetzung auf eine PTFE-beschichtete Folie zu einem Film von 2 mm Dicke ausgegossen, während 7 Tagen im Normklima gelagert, einige Hanteln mit einer Länge von 75 mm bei einer Steglänge von 30 mm und einer Stegbreite von 4 mm aus dem Film ausgestanzt und diese gemäss DIN EN 53504 bei einer Zuggeschwindigkeit von 200 mm/min auf **Zugfestigkeit** (Bruchkraft), **Bruchdehnung, E-Modul 5%** (bei 0.5-5% Dehnung) und **E-Modul 50%** (bei 0.5-50% Dehnung) geprüft.

Der **Aspekt** wurde visuell an den hergestellten Filmen beurteilt. Als "schön" wurde ein klarer Film mit nichtklebriger Oberfläche ohne Blasen bezeichnet. Der **Geruch** wurde durch Riechen mit der Nase im Abstand von 2 cm an den frisch hergestellten Filmen beurteilt. "Nein" bedeutet, dass kein Geruch wahrnehmbar war.

Die Resultate sind in der Tabelle 2 angegeben.

Bei den Zusammensetzungen **Z1** bis **Z3** handelt es sich um erfindungsgemässe Beispiele. Zusammensetzungen **Ref1** bis **Ref3** sind Vergleichsbeispiele.

**Tabelle 2: Zusammensetzung (in Gewichtsteilen) und Eigenschaften von Z1 bis Z3 und Ref1 bis Ref3.**

| **Zusammensetzung** | | **Z1** | **Ref1** | **Z2** | **Ref2** | **Z3** | **Ref3** |
|---|---|---|---|---|---|---|---|
| Umsetzungsprodukt | | **U1** | **Q1** | **U2** | **Q2** | **U4** | **Q4** |
| | | 80.00 | 80.00 | 80.00 | 80.00 | 80.00 | 80.00 |
| Salicylsäurelösung¹ | | 1.50 | 0.50 | 1.50 | 0.50 | 1.50 | 0.50 |
| Viskosität (1d RT) | | 201 | 77 | 73 | 82 | 121 | 137 |
| | [Pa·s] (7d 60°C) | 371 | 94 | 98 | 104 | 148 | 180 |
| tack-free time | | 195' | 400' | 165' | 195' | 45' | 45' |
| Zugfestigkeit [MPa] | | 3.41 | 5.51 | 1.16 | 0.60 | 0.57 | 0.69 |
| Bruchdehnung [%] | | 946 | >1200 | >1200 | 494 | >1200 | >1200 |
| E-Modul 5% [MPa] | | 1.33 | 1.32 | 0.85 | 0.91 | 0.76 | 0.58 |
| E-Modul 50% | | 0.70 | 0.67 | 0.48 | 0.53 | 0.39 | 0.28 |
| Aspekt | | schön | schön | schön | schön | schön | schön |
| Geruch | | nein | nein | nein | nein | nein | nein |

| | | | | | | | |
|---|---|---|---|---|---|---|---|
| ¹ 5% in Dioctyladipat | | | | | | | |

### Zusammensetzungen Z4 und Z5 und Ref4

Für jede Zusammensetzung wurden die in der Tabelle 3 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt und unter Feuchtigkeitsausschluss aufbewahrt. Jede Zusammensetzung wurde folgendermassen geprüft:
Als Mass für die Weichmacher-Migration wurde jede Zusammensetzung so auf eine Kartonunterlage appliziert, dass sie eine runde Grundfläche von 12 mm Durchmesser und eine Höhe von 20 mm aufwies, und während 7 Tagen im NK gelagert. Um jede Zusammensetzung herum war danach auf dem Karton ein dunkler ovaler Fleck entstanden. Dessen Ausmasse (Höhe und Breite) wurden ausgemessen und in der Tabelle 3 als **Migration** angegeben.

Bei den Zusammensetzungen **Z4** und **Z5** handelt es sich um erfindungsgemässe Beispiele. Zusammensetzung **Ref4** ist ein Vergleichsbeispiel.

**Tabelle 3: Zusammensetzung (in Gewichtsteilen) und Eigenschaften von Z4 und Z5 und Ref4.**

| **Zusammensetzung** | | **Z4** | **Z5** | **Ref4** |
|---|---|---|---|---|
| Umsetzungsprodukt | | **U7** | **U8** | **Q7** |
| | | 25.0 | 25.0 | 25.0 |
| Kreide¹ | | 25.0 | 25.0 | 25.0 |
| Kieselsäure² | | 1.9 | 1.9 | 1.9 |
| Dibutylzinndilaurat³ | | 2.5 | 2.5 | 2.5 |
| Salicylsäure⁴ | | 5.0 | 5.0 | 5.0 |
| Migration [mm] | Höhe | 1 | 1 | 8 |
| | Breite | 1 | 1 | 6 |

| | | | | |
|---|---|---|---|---|
| ¹ gemahlenes, mit Fettsäure beschichtetes Calciumcarbonat ² hydrophob modifizierte pyrogene Kieselsäure ³ 5% in Diisodecylphthalat ⁴ 5% in Dioctyladipat | | | | |

### Zweikomponentige Zusammensetzungen

### Zusammensetzungen Z6 und Z7 und Ref5 und Ref6

Für jede Zusammensetzung wurden die in der Tabelle 4 angegebenen Inhaltsstoffe in den angegebenen Mengen (in Gewichtsteilen) der ersten Komponente K1 mittels eines Zentrifugalmischers (SpeedMixer^{™} DAC 150, FlackTek Inc.) vermischt und unter Ausschluss von Feuchtigkeit aufbewahrt.

Als zweite Komponente K2 wurde Desmodur^{®} N 3300 (HDI-Trimer mit einem NCO-Gehalt von 21.8 Gewichts-%, von Covestro) in der in der Tabelle 4 angegebenen Menge (in Gewichtsteilen) verwendet.

Anschliessend wurden die beiden Komponenten jeder Zusammensetzung mittels Zentrifugalmischer zu einer homogenen Flüssigkeit verarbeitet und diese unverzüglich geprüft, wie für die Zusammensetzung **Z1** beschrieben.

Die Resultate sind in der Tabelle 4 angegeben.

Bei den Zusammensetzungen **Z6** und **Z7** handelt es sich um erfindungsgemässe Beispiele. Zusammensetzungen **Ref5** und **Ref6** sind Vergleichsbeispiele.

**Tabelle 4: Zusammensetzung (in Gewichtsteilen) und Eigenschaften von Z6 und Z7 und Ref5 und Ref6.**

| **Zusammensetzung** | | | **Z6** | **Ref5** | **Z7** | **Ref6** |
|---|---|---|---|---|---|---|
| **Komponente K1:** | | | | | | |
| | | | **U5** | **Q5** | **U6** | **Q6** |
| | | Umsetzungsprodukt | 75.00 | 75.00 | 75.00 | 75.00 |
| | | Salicylsäurelösung¹ | 1.50 | 0.50 | 1.50 | 0.50 |

| **Komponente K2:** | | | | | | |
|---|---|---|---|---|---|---|
| | | Desmodur^{®} N 3300 | 6.83 | 6.79 | 6.67 | 6.74 |
| | tack-free time | | 55' | 45' | 70' | 70' |
| | Zugfestigkeit [MPa] | | 1.60 | 1.80 | 1.66 | 1.88 |
| | Bruchdehnung [%] | | 127 | 150 | 161 | 202 |
| | E-Modul 5% [MPa] | | 2.29 | 2.32 | 1.15 | 0.72 |
| | E-Modul 50% [MPa] | | 1.53 | 1.51 | 0.85 | 0.54 |
| | Aspekt | | schön | schön | schön | schön |
| | Geruch | | nein | nein | nein | nein |

| | | | | | | |
|---|---|---|---|---|---|---|
| ¹ 5% in Dioctyladipat | | | | | | |

### Reaktive Heissschmelzklebstoffe

### Klebstoffe H1 und HR1 und HR2:

Eine Mischung aus 30.6 g Polyoxypropylendiol (OH-Zahl 56.0 mg KOH/g), 7.2 g Polyoxypropylendiol (OH-Zahl 112.5.0 mg KOH/g) und 23.4 g bei Raumtemperatur festes Acrylatpolymer (Copolymer von Methylmethacrylat und n-Butylmethacrylat) wurde unter Vakuum unter Rühren auf 140°C aufgeheizt, bis das Acrylatpolymer vollständig gelöst war. Dann wurden 18.0 g bei Raumtemperatur kristallines, lineares Polyesterdiol (aus Hexandiol und Adipinsäure; OH-Zahl 28.0 mg KOH/g) zugegeben und die Mischung während 1 h bei 140°C unter Vakuum gerührt. Anschliessend wurden 13.6 g 4,4'-Diphenylmethandiisocyanat (Desmodur^{®} 44 MC L, von Covestro) zugegeben und während 30 Minuten bei 140 °C unter Vakuum gerührt. Dann wurden entweder 7.2 g **Hydroxyaldimin A1 (Klebstoff H1)** oder 7.2 g **Hydroxyaldimin R1 (Klebstoff HR1)** oder nichts **(Klebstoff HR2)** zugegeben und während weiteren 30 Minuten bei 140°C unter Vakuum gerührt, bevor die Mischung abgekühlt und unter Ausschluss von Feuchtigkeit aufbewahrt wurde.

Jeder Klebstoff wurde folgendermassen geprüft:
Der **Gehalt an 4,4'-MDI** wurde bestimmt mittels HPLC (Detektion über Photodiodenarray; 0.04 M Natriumacetat / Acetonitril als mobile Phase).

Die **Viskosität** wurde am Folgetag der Herstellung mit Brookfield Thermosel bei 5 rpm mit Spindel 27 bei der angegebenen Temperatur gemessen und als "Viskosität (frisch)" bezeichnet.

Zur Bestimmung der Frühfestigkeit und der mechanischen Eigenschaften wurde der jeweilige Heissschmelzklebstoff bei 150°C auf Silikonpapier appliziert und abkühlen lassen, so dass ein Film von 500 µm Dicke entstand. Daraus wurden Prüfkörper von 20 x 100 mm ausgeschnitten und im Normklima gelagert. Nach 30 min wurde ein erstes Mal die **Zugfestigkeit** bestimmt (Mass für die Frühfestigkeit), dann erneut nach 14 Tagen die Zugfestigkeit und die **Bruchdehnung,** jeweils in Anlehnung an DIN EN 53504 bei einer Zuggeschwindigkeit von 10 mm/min.

Zur Bestimmung der **thermischen Stabilität** im geschmolzenen Zustand bei Umgebungsfeuchte wurde der Klebstoff im Normklima auf einer bei 140°C temperierten offenen Walze rotieren lassen. In regelmässigen Zeitabständen wurde die Viskosität (Brookfield Thermosel, 5 rpm, Spindel 27) gemessen.

Die Resultate sind in der Tabelle 5 angegeben.

Bei Klebstoff **H1** handelt es sich um ein erfindungsgemässes Beispiel. Die **Klebstoffe HR1** und **HR2** sind Vergleichsbeispiele.

**Tabelle 5: Eigenschaften der Klebstoffe H1, HR1 und HR2.**

| **Klebstoff** | | **H1** | **HR1** | **HR2** |
|---|---|---|---|---|
| zugegebenes Hydroxyaldimin | | **A1** | **R1** | **-** |
| Gehalt an 4,4'-MDI | | 0.28 Gew.-% | 0.12 Gew.-% | 1.90 Gew.-% |
| Viskosität (frisch) bei 140 °C | | 0.3 Pa·s | 3.5 Pa·s | 0.5 Pa.s |
| Zugfestigkeit (nach 30') | | 0.35 MPa | 0.61 MPa | <0.3 MPa |
| Zugfestigkeit (nach 14d) | | 6.9 MPa | 6.0 MPa | n.d.* |
| Bruchdehnung (nach 14d) | | 590% | 530% | n.d.* |
| Viskosität (140 °C) nach | | | | |
| | 0.5 h | 0.3 Pa·s | 3.7 Pa·s | 0.5 Pa·s |
| | 1.0 h | 0.3 Pa·s | 4.4 Pa·s | n.d. |
| | 1.5 h | 0.3 Pa·s | 6.8 Pa·s | n.d. |
| | 2.0 h | 0.3 Pa·s | 7.1 Pa·s | n.d. |
| | 2.5 h | 0.3 Pa·s | 7.6 Pa·s | n.d. |
| | 3.0 h | 0.3 Pa·s | 8.0 Pa·s | n.d. |
| | 3.5 h | 0.3 Pa·s | 12.4 Pa·s | n.d. |
| | 4.0 h | 0.3 Pa·s | 16.6 Pa·s | n.d. |
| | 4.5 h | 0.3 Pa·s | 24.7 Pa·s | 0.6 Pa·s |
| | 5.0 h | 0.3 Pa·s | n.m. | n.d. |
| | 8.0 h | 0.3 Pa·s | n.m. | n.d. |
| | 9.0 h | 0.3 Pa·s | n.m. | n.d. |
| | 10.0 h | 2.0 Pa·s | n.m. | n.d. |
| | 12.0 h | 4.7 Pa·s | n.m. | n.d. |
| | 14.0 h | 5.4 Pa·s | n.m. | n.d. |
| | 16.0 h | 4.8 Pa·s | n.m. | 2.2 Pa·s |

| | | | | |
|---|---|---|---|---|
| "n.m." steht für "nicht messbar" (zu hochviskos/geliert). "n.d." steht für "nicht gemessen". *Blasen im Film | | | | |

## Patentansprüche

1. Hydroxyaldimin der Formel (I), 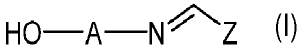 wobei
Z für einen mit einer Alkyl- und/oder Alkoxy-Gruppe substituierten Aryl-Rest mit insgesamt 12 bis 26 C-Atomen steht, und
A für einen zweiwertigen aliphatischen oder cycloaliphatischen oder arylaliphatischen, gegebenenfalls Ether-Sauerstoff aufweisenden Kohlenwasserstoff-Rest mit einem Molekulargewicht im Bereich von 28 bis 500 g/mol steht.

2. Hydroxyaldimin gemäss Anspruch 1, **dadurch gekennzeichnet, dass** Z für für einen Rest der Formel (II) steht, 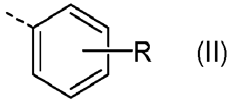 wobei R für einen linearen oder verzweigten Alkyl- oder Alkoxy-Rest mit 6 bis 20 C-Atomen steht.

3. Hydroxyaldimin gemäss Anspruch 2, **dadurch gekennzeichnet, dass** R verzweigt ist.

4. Hydroxyaldimin gemäss Anspruch 3, **dadurch gekennzeichnet, dass** R für einen Rest der Formel 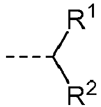 steht, wobei R¹ und R² jeweils für einen Alkyl-Rest stehen und zusammen 9 bis 13 C-Atome aufweisen.

5. Hydroxyaldimin gemäss einem der Ansprüche 1 bis 4, **dadurch gekennzeichnet, dass** A ausgewählt ist aus der Gruppe bestehend aus 1,2-Ethylen, 1,2-Propylen, 1,3-Propylen, 1,5-Pentylen, 1,6-Hexylen, (1,5,5-Trimethylcyclohexan-1-yl)methan-1,3 und 3-Oxa-1,5-pentylen.

6. Reaktionsprodukt enthaltend mindestens ein Hydroxyaldimin gemäss einem der Ansprüche 1 bis 5, erhalten aus der Umsetzung von mindestens einem Amin der Formel (III) mit mindestens einem Aldehyd der Formel (IV) in einer Kondensationsreaktion unter Freisetzung von Wasser, wobei der Aldehyd stöchiometrisch oder im stöchiometrischen Überschuss in Bezug auf die primären Aminogruppen vorhanden war.

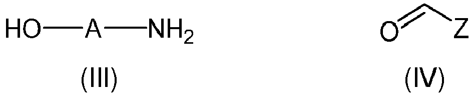

7. Gemisch aus Hydroxyaldiminen gemäss einem der Ansprüche 1 bis 6, bei welchen Z jeweils für einen Rest der Formel (II) steht und R dabei ausgewählt ist aus linearen oder insbesondere verzweigten Decyl-, Undecyl-, Dodecyl-, Tridecyl- und Tetradecyl-Resten.

8. Umsetzungsprodukt mit Aldiminogruppen der Formel (V), 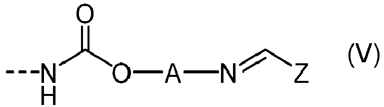 erhalten aus der Umsetzung von mindestens einem Hydroxyaldimin gemäss einem der Ansprüche 1 bis 7 mit mindestens einem Polyisocyanat.

9. Umsetzungsprodukt gemäss Anspruch 8, **dadurch gekennzeichnet, dass** es zusätzlich Isocyanatgruppen aufweist und das OH/NCO-Verhältnis bei der Umsetzung im Bereich von 0.05/1 bis 0.5/1 liegt.

10. Umsetzungsprodukt gemäss Anspruch 8, **dadurch gekennzeichnet, dass** es frei ist von Isocyanatgruppen und das OH/NCO-Verhältnis bei der Umsetzung im Bereich von 1/1 bis 1.5/1 liegt.

11. Isocyanatgruppen-haltige Zusammensetzung enthaltend mindestens ein Umsetzungsprodukt gemäss einem der Ansprüche 8 bis 10.

12. Zusammensetzung gemäss Anspruch 11, **dadurch gekennzeichnet, dass** sie zusätzlich mindestens einen weiteren Bestandteil ausgewählt aus Katalysatoren, Füllstoffen, Weichmachern und Lösemitteln enthält.

13. Zusammensetzung gemäss einem der Ansprüche 11 oder 12, **dadurch gekennzeichnet, dass** sie einen Klebstoff oder einen Dichtstoff oder eine Beschichtung darstellt.

14. Zusammensetzung gemäss Anspruch 13, **dadurch gekennzeichnet, dass** sie einen reaktiven Heissschmelzklebstoff darstellt.

15. Verfahren zur Reduktion des Gehaltes an monomeren Diisocyanaten von einem Isocyanatgruppen-haltigen Polyurethanpolymer, indem dieses in einem unterstöchiometrischen OH/NCO-Verhältnis unter Ausschluss von Feuchtigkeit mit mindestens einem Hydroxyaldimin gemäss einem der Ansprüche 1 bis 7 umgesetzt wird.
